# EUROPEAN PATENT APPLICATION

(11) **EP 4 410 816 A1**
(43) Date of publication of application: **07.08.2024**
(21) Application number: 22876574.9
(22) Date of filing: 30.09.2022
(51) Int. Cl.: C07K 7/08, A61K 38/10, A61K 38/12, A61P 9/10, A61P 13/12, A61P 35/00, A61P 43/00, C07K 7/54

(54) **PEPTIDE**

(30) Priority: 30.09.2021 JP 2021161353
(71) Applicant: PeptiDream Inc., Kawasaki-shi, Kanagawa 210-0821 (JP)
(72) Inventor: SUZUKI, Yoshinori, Kawasaki-shi, Kanagawa 210-0821 (JP); KINEBUCHI, Masahiko, Kawasaki-shi, Kanagawa 210-0821 (JP)
(74) Representative: Mewburn Ellis LLP
(86) International application number: PCT/JP2022/036850
(87) International publication number: WO 2023/054712

(57) **Abstract**

The present invention relates to a peptide and a composition containing the peptide. This peptide of the present invention comprises the following amino acid sequence: F-Nal1-V-V-N-V-Y-D-D-PeG-V-Nal1-Y-H-V-C-G (SEQ ID NO: 2); or an amino acid sequence having a substitution, addition, deletion, or insertion in 1 to 11 amino acid residues selected from the group consisting of amino acid residues at positions 2, 4, 7, 8, 9, 10, 12, 13, 14, 15, and 17 in the above amino acid sequence.

## Description

### TECHNICAL FIELD

The present invention relates to a peptide, a composition containing the peptide, and use of the peptide.

### BACKGROUND ART

TGF-β (transforming growth factor β) is a cytokine belonging to the TGF-β family and has many functions. The TGF-β family includes three isoforms of TGF-β (TGF-β1, β2 and β3) and many other signal transduction proteins. TGF-β is known to have multiple functions, such as regulating cell proliferation and differentiation, and promoting the production and deposition of extracellular matrix proteins (e.g., collagen, fibronectin). TGF-β-mediated signal transduction pathways are known to be involved in, for example, cancer cell proliferation, metastasis, angiogenesis, and regulation of immune responses (JP 2021-100972 A). Therefore, research and development of substances affecting the TGF-β functions, especially compounds that inhibit or weaken TGF-β signal transduction (e.g., TGF-β antagonists) has been sought for medicament. Since the TGF-β signaling pathway substantially affects cell proliferation, survival, and differentiation, the substances have been studied as an additive to medium.

Substances with TGF-β antagonist activity, such as antibodies (WO 01/066140, WO 2012/030394) and small molecules (WO 2015/103355), are known. They are being investigated, for example, as therapeutic agents for renal dysfunction and/or myocardial infarction using these substances. In recent years, the immunosuppressive effect of TGF-β on tumor has been attracting attention, and the combination of an immune checkpoint inhibitor and a TGF-β neutralizing antibody has also been studied.

However, there are no reports on any peptide with TGF-β binding activity and TGF-β antagonist activity.

### CITATION LIST

### PATENT LITERATURE

PTL 1: JP 2021-100972 A
PTL 2: WO 01/066140
PTL 3: WO 2012/030394
PTL 4: WO 2015/103355

### NON PATENT LITERATURE

NPL 1: Massague, Nat. Rev. Mol. Cell Biol., 2012; 13(10)
NPL 2: Zhang et al., Cold Spring Harb Perspect Biol., 2017 ;9(4)

### SUMMARY OF INVENTION

### TECHNICAL PROBLEM

The present inventors have conducted intensive research to create a peptide having TGF-β binding activity and preferably TGF-β antagonist activity, and as a result, have conceived the present invention. The present invention provides a peptide, a composition containing the peptide, and use of the peptide.

### SOLUTION TO PROBLEM

Although not limited, the present application includes the following items of the invention.
[1] A peptide comprising the following amino acid sequence:
   F-Nal1-V-V-N-V-Y-D-D-PeG-V-Nal1-Y-H-V-C-G (SEQ ID NO: 2); or an amino acid sequence having a substitution, addition, deletion, or insertion in 1 to 11 amino acid residues selected from the group consisting of amino acid residues at positions 2, 4, 7, 8, 9, 10, 12, 13, 14, 15, and 17 in the above amino acid sequence.
[2] A peptide comprising the following amino acid sequence:
   F-X1-V-X2-N-V-X3-X4-X5-X6-V-X7-X8-X9-X10-C (SEQ ID NO: 1)
   wherein
   X1 is an amino acid having an optionally substituted aromatic ring in a side chain;
   X2 is any amino acid;
   X3 is any amino acid;
   X4 is any amino acid;
   X5 is any amino acid;
   X6 is an amino acid having an optionally substituted alkyl group in a side chain or a secondary amino acid having an optionally substituted alkyl group;
   X7 is an amino acid having an optionally substituted aromatic ring in a side chain;
   X8 is an amino acid having an optionally substituted aromatic ring in a side chain;
   X9 is any amino acid; and
   X10 is any amino acid.
[3] The peptide according to [2], wherein X1 is an amino acid having an optionally substituted fused ring in a side chain.
[4] The peptide according to [2], wherein X1 is Nal1, W6N, W7N, or W.
[5] The peptide according to any one of [2] to [4], wherein X2 is V, K, KCOpipzaa, KCOmeglumine, or Q.
[6] The peptide according to any one of [2] to [4], wherein X2 is V, K, KCOpipzaa, or KCOmeglumine.
[7] The peptide according to any one of [2] to [6], wherein X3 is Y, 4Py, A, E, F4G, Q,K, F3G, or 3Py.
[8] The peptide according to any one of [2] to [6], wherein X3 is Y or 4Py.
[9] The peptide according to any one of [2] to [8], wherein X4 is an amino acid other than a substituted or unsubstituted aromatic or sulfur-containing amino acid.
[10] The peptide according to any one of [2] to [8], wherein X4 is D, A, Q, K, or E.
[11] The peptide according to any one of [2] to [10], wherein X5 is an amino acid other than a substituted or unsubstituted aromatic or sulfur-containing amino acid.
[12] The peptide according to any one of [2] to [10], wherein X5 is D, E, KCOpipzaa, KCOmeglumine, A, Q, or K.
[13] The peptide according to any one of [2] to [12], wherein X6 is PeG, K, or MeG.
[14] The peptide according to any one of [2] to [13], wherein X7 is an amino acid having an optionally substituted fused ring in a side chain.
[15] The peptide according to any one of [2] to [13], wherein X7 is Nal1, W6N, or W.
[16] The peptide according to any one of [2] to [15], wherein X8 is Y, 4Py, 3Py, or E.
[17] The peptide according to any one of [2] to [15], wherein X8 is Y or 4Py.
[18] The peptide according to any one of [2] to [17], wherein X9 is an amino acid other than a substituted or unsubstituted aromatic or sulfur-containing amino acid.
[19] The peptide according to any one of [2] to [17], wherein X9 is H, A, Q, or E.
[20] The peptide according to any one of [2] to [19], wherein X10 is an amino acid other than a substituted or unsubstituted aromatic or sulfur-containing amino acid.
[21] The peptide according to any one of [2] to [19], wherein X10 is V, E, KCOpipzaa, KCOmeglumine, Q, K, or A.
[22] The peptide according to any one of [1] to [21], wherein the peptide comprises or consists of an amino acid sequences set forth in any one of
   SEQ ID NOs: 2 to 33 or
   SEQ ID NOs: 2, 4-13, and 15-33 without a C-terminal glycine (G).
[23] The peptide according to any one of [1] to [22], wherein the peptide is a cyclic peptide.
[24] The cyclic peptide according to [23], which has a cyclic structure in which a chloroacetylated amino acid is bonded to a cysteine residue included in the peptide.
[25] The peptide according to any one of [1] to [24], further comprising an additional amino acid residue.
[26] The peptide according to any one of [1] to [21] and [23] to [25], comprising a linker at a C-terminus thereof.
[27] The peptide according to any one of [1] to [26], having TGF-β binding activity.
[28] The peptide according to any one of [1] to [27], having TGF-β antagonist activity.
[29] A medical or diagnostic composition comprising the peptide according to any one of [1] to [28].
[30] A research composition comprising the peptide according to any one of [1] to [28], excluding a composition used as an additive to a medium for culturing an organoid.

### ADVANTAGEOUS EFFECTS OF INVENTION

The peptide of the present invention has TGF-β binding activity and preferably TGF-β antagonist activity, and is useful as a pharmaceutical composition (e.g., for cancer, and liver diseases), a diagnostic composition, and a research composition.

### BRIEF DESCRIPTION OF DRAWING

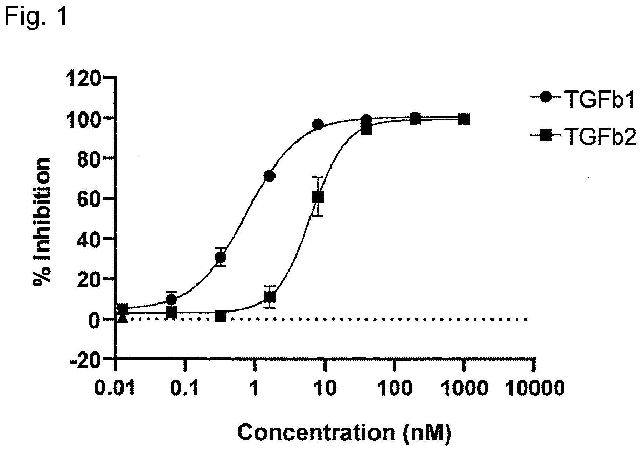

[Fig. 1] Fig. 1 shows the results of measuring inhibitory activity of TGFb1_012 (SEQ ID NO: 13) against TGF-β1 or TGF-β2 by SBE reporter assay. In Fig. 1, black circles indicate TGF-β1 and black squares indicate TGF-β2. The horizontal axis indicates the concentration (nM) of TGF-β1_012 (SEQ ID NO: 13), and the vertical axis indicates the inhibitory activity.

### DESCRIPTION OF EMBODIMENTS

### 1. Abbreviations

As used herein, the following abbreviations are used with the following meanings unless otherwise indicated.

### Abbreviations (general)

Å: angstrom (unit);
ClAc: chloroacetyl;
DCM: dichloromethane;
DMSO: dimethyl sulfoxide;
DMF: dimethylformamide;
DIEA or DIPEA: N,N-diisopropylethylamine;
DIPCI: N,N'-diisopropylcarbodiimide;
DODT: 6-dioxa-1,8-octane-dithiol;
Fmoc: 9-fluorenylmethyloxycarbonyl;
NHS: N-Hydroxysuccinimide;
g: gram (unit);
HATU: O-(7-azabenzotriazol-1-yl)-N,N,N',N'-tetramethyluronium hexafluorophosphate;
HOSu: N-Hydroxysuccinimide;
HPLC: high-performance liquid chromatography;
LC-MS or LC/MS: liquid chromatography mass spectrometer;
mL: milliliter (unit);
M: molar (unit);
µL: microliter (unit);
mM: millimolar (unit);
µM: micromolar (unit);
mg: milligram (unit);
MeCN: acetonitrile;
mm: millimeter (unit);
µm: micrometer (unit);
nM: nanomolar (unit);
OSu: succinimide;
PEG: polyethylene glycol;
rpm: revolutions per min (unit);
tBu: *tert*-butyl;
Mpe: methylpentan-3-yl;
Boc: tert-butoxycarbonyl;
TFA: trifluoroacetic acid;
TIS: triisopropylsilane;
Trt or Tr: trityl group;
TIPS: triisopropylsilyl group.

### Abbreviations (non-natural amino acids)

Nal1: 1-naphthyl-L-alanine (CAS No. 55516-54-6);

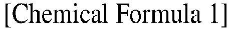

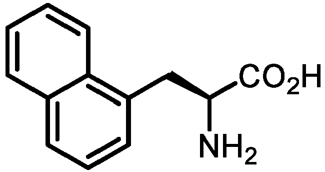

W6N: 6-aza-L-tryptophan (CAS No. 149704-63-2); 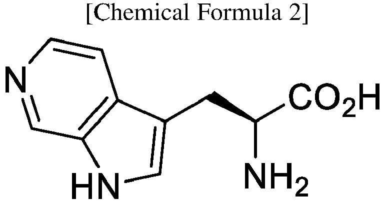
W7N: L-7-azatryptophan (CAS No. 49758-35-2); 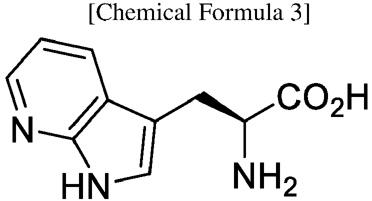
KCOpipzaa: N6-(4-(carboxymethyl)piperazin-1-carbonyl)-L-lysine (CAS No. N/A); 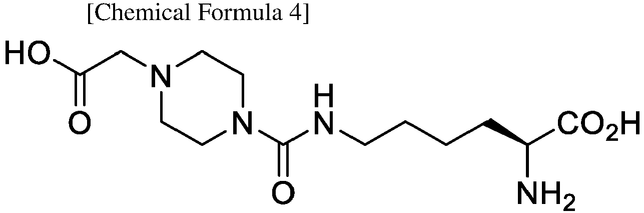
KCOmeglumine: N6-(methyl((2S,3R,4R,5R)-2,3,4,5,6-pentahydroxyhexyl)carbamoyl)-L-lysine; 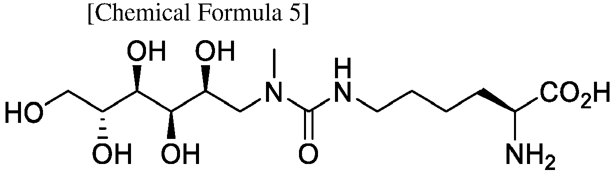
4Py: 4-pyridyl-L-alanine (CAS No. 37535-49-2); 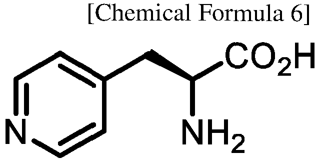
PeG: phenethylglycine (CAS No. 7738-38-7); 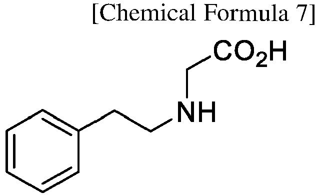
F4G: p-guanidino-L-phenylalanine (CAS No. 59574-11-7); 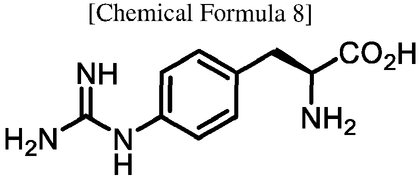
F3G: 3-[(aminoiminomethyl)amino]-L-phenylalanine (CAS No. 1019057-42-1); 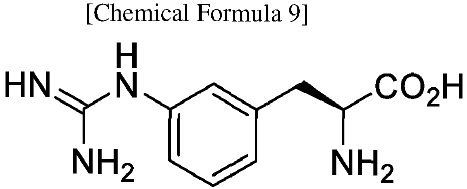
3Py: 3-(3-pyridyl)-L-alanine (CAS No. 64090-98-8); 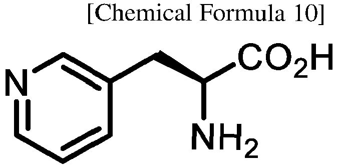
MeG: methylglycine (CAS No. 107-97-1); 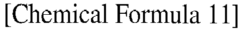 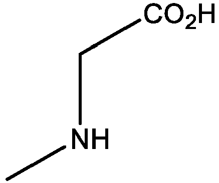
PEG10c: 1-amino-3,6,9,12,15,18,21,24,27,30-decaoxatri-triacontan-33-oic acid;
MePEG8c: 2,5,8,11,14,17,20,23-octaoxahexacosan-26-oic acid;
OCOmPEG10000: methoxy polyethylene glycol carbonate (average molecular weight 10,000).

### 2. Peptide (A)

The present invention relates to peptides.

An embodiment of the present invention provides a peptide comprising the following amino acid sequence:
F-Nal1-V-V-N-V-Y-D-D-PeG-V-Nal1-Y-H-V-C-G (SEQ ID NO: 2);
or an amino acid sequence having a substitution, addition, deletion, or insertion in 1 to 11 amino acid residues selected from the group consisting of amino acid residues at positions 2, 4, 7, 8, 9, 10, 12, 13, 14, 15, and 17 in the above amino acid sequence.

SEQ ID NO: 2 is a peptide which has been confirmed to have TGF-β binding activity and TGF-β antagonist activity in the Examples herein.

The amino acid sequence may have a substitution, addition, deletion, or insertion in 1 to 11 amino acid residues selected from the group consisting of amino acid residues at positions 2, 4, 7, 8, 9, 10, 12, 13, 14, 15, and 17 in SEQ ID NO: 2. The number of amino acid substitutions, deletions, additions, and/or insertions should be 1 or more and 10 or less and the lower limit is 1. The upper limit may be 10, 9, 8, 7, 6, 5, 4, 3, or 2, and the minimum is 1. Preferred is an amino acid "substitution". Such an amino acid substitution is preferably a conservative amino acid substitution.

The term "conservative amino acid substitution" means a substitution with a functionally equivalent or similar amino acid. A conservative amino acid substitution in a peptide causes a static change in the amino acid sequence of the peptide. For example, one or two or more amino acids with similar polarity act in a functionally equivalent manner to produce a static change in the amino acid sequence of such a peptide. In general, a substitution within a group can be considered structurally and functionally conservative. However, as obvious to those skilled in the art, the role played by a particular amino acid residue can be determined by its significance in the three-dimensional structure of the molecule containing that amino acid. For example, a cysteine residue can take a less polar, oxidized (disulfide) form when compared to the reduced (thiol) form. The long aliphatic portion of an arginine side chain can constitute a structurally and functionally important feature. In addition, a side chain having an aromatic ring (of tryptophan, tyrosine, or phenylalanine) can contribute to an ion-aromatic or cation-pi interaction. In such cases, substitution of an amino acid having such a side chain with an amino acid belonging to an acidic or nonpolar group can be structurally and functionally conservative. A residue such as proline, glycine, or cysteine (disulfide form) can have a direct effect on the conformation of the main chain and often cannot be replaced without any structural distortion.

The conservative amino acid substitution includes a specific substitution based on side chain similarity (e.g., L. Lehninger, Biochemistry, 2nd edition, pp73-75, Worth Publisher, New York (1975)) and a typical substitution as shown below.

In addition, for example, the conservative amino acid substitution is preferably a substitution to an amino acid belonging to the same group as a group of amino acids as grouped on the basis of side chain properties common to those of the following natural amino acids.

Hydrophobic (also called nonpolar) amino acids: amino acids that are hydrophobic (nonpolar) and include alanine ("Ala" or simply "A"), glycine ("Gly" or simply "G"), valine ("Val" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W"), tyrosine ("Tyr" or simply "Y"), and methionine ("Met" or simply "M").

Note that the hydrophobic amino acids can be further divided into the following groups.

Aliphatic amino acids: amino acids with fatty acid or hydrogen in the side chain, including Ala, Gly, Val, Ile, and Leu.

Aliphatic/branched-chain amino acids: amino acids with branched fatty acid in the side chain, including Val, Ile, and Leu.

Aromatic amino acids: amino acids with an aromatic ring in the side chain, including Trp, Tyr, and Phe.

Hydrophilic (also called polar) amino acids: amino acids that are hydrophilic (polar) and include serine (Ser" or simply "S"), threonine ("Thr" or simply "T"), cysteine ("Cys" or simply "C"), asparagine ("Asn" or simply "N"), glutamine ("Gln" or simply "Q"), aspartic acid ("Asp" or simply "D"), glutamic acid ("Glu" or simply "E"), and lysine ("Lysine"; "Lys" or simply "K"), arginine ("Arg" or simply "R"), and histidine ("His" or simply "H").

Note that the hydrophilic amino acids can be further divided into the following groups.

Acidic amino acids: amino acids with an acidic side chain, including Asp and Glu.

Basic amino acids: amino acids with a basic side chain, including Lys, Arg, and His.

Neutral amino acids: amino acids with a neutral side chain, including Ser, Thr, Asn, Gln, and Cys.

Meanwhile, Gly and Pro can be classified as "amino acids affecting the direction of the main chain", and amino acids containing a sulfur molecule in the side chain, Cys and Met can also be classified as "sulfur-containing amino acids".

In addition, examples of the group having an aromatic group in a side chain include Trp, Tyr, and Phe.

As used herein, the "amino acids" include not only natural amino acids but also non-natural amino acids. Examples of the non-natural amino acids include N-alkylamino acids, in which the above-described natural amino acid is N-alkylated, and those in which the nitrogen forming a peptide bond is modified with a branched or unbranched lower (e.g., C1-C5, preferably C1-C3, more preferably C1) alkyl group. The N-alkylamino acids are preferably N-ethylamino acids, N-butylamino acids, or N-methylamino acids, and more preferably N-methylamino acids. In addition, examples of the non-natural amino acids include D-type amino acids (also called D-amino acids), β-amino acids, γ-amino acids, amino acid mutants, chemically modified amino acids such as amino acid derivatives, and amino acids, such as norleucine and ornithine, that are not used as building blocks of proteins *in vivo.* Further, the examples include amino acids in which the side chain of a natural amino acid further contains a functional group or is substituted by another functional group (e.g., amino acids with, for example, a side chain arylene or alkylene group moiety with substitution and/or addition, amino acids with an increased number of carbon atoms in an arylene, alkylene or alkyl group in the side chain, amino acids with a substituted aromatic ring in a side chain, amino acid derivatives having a structure in which an amino acid having a carboxylic acid functional group in a side chain is substituted with a sulfonic acid group, heterocyclized, fused-ring amino acids, etc.).

Note that a nature amino acid side chain may contain or be substituted with a structure such as a functional group. This can impart a property different from that of a natural amino acid. In other words, non-natural amino acids with similar side chain properties can be included in the above-described groups obtained by classifying natural amino acids based on their common side chain properties. As such, non-natural amino acids that exhibit side-chain properties similar to those of a certain amino acid can also be included as targets for conservative amino acid substitution.

Examples of the non-natural amino acid include, but are not limited to, N-methylamino acid, 4Py, Nal1, W6N, W7N, KCOpipzaa, KCOmeglumine, or PeG.

For example, Nal1 and PeG can be grouped into hydrophobic amino acids and 4Py, W7N and W6N can be grouped into hydrophilic amino acids. Further, 4Py, W7N, and W6N can be grouped into basic amino acids and 4Py, Nal1, PeG, W6N, and W7N can be grouped into aromatic amino acids. Note that N-methylamino acids can be classified as N-alkylamino acids or classified according to the nature of the side chain of the original amino acid that is not N-methylated.

D-amino acids can be classified as D-amino acids or classified according to the nature of their side chains. N-methylamino acids can be classified as N-alkylamino acids or classified according to the nature of the side chain of the original amino acid that is not N-methylated.

The peptide is not limited and a cyclic peptide in one embodiment. The "cyclic peptide" is described in detail in "3. Peptide (B)". As used herein, "not limited" and "one embodiment" may be used synonymously.

In one embodiment, the peptide may comprise an additional amino acid residue(s) in addition to SEQ ID NO: 2. The "additional amino acid residue(s)" is described in detail in "3. Peptide (B)".

In one embodiment, the above peptide may comprise a linker, preferably at the C-terminus. The "linker" is described in detail in "3. Peptide (B)". The "G" at the C-terminus (17th amino acid residue) of SEQ ID NO: 2 is a linker. In one embodiment, the peptide may have a linker other than G (e.g., a PEG linker). Alternatively, another linker may be further included in addition to G at the C-terminus. For example, an embodiment of such as glycine-rich peptide is also included.

The peptide preferably has TGF-β binding activity and more preferably has TGF-β1 binding activity. The above peptide preferably has TGF-β antagonist activity. The details of "having TGF-β1 binding activity" and "having TGF-β antagonist activity" are described in "3. Peptide (B)".

The "peptide" herein includes both peptide (A) and peptide (B) unless otherwise indicated.

### 3. Peptide (B)

The present invention relates to peptides.

The present invention a peptide comprising the following amino acid sequence:
F-X1-V-X2-N-V-X3-X4-X5-X6-V-X7-X8-X9-X10-C (SEQ ID NO: 1) wherein
X1 is an amino acid having an optionally substituted aromatic ring in a side chain;
X2 is any amino acid;
X3 is any amino acid;
X4 is any amino acid;
X5 is any amino acid;
X6 is an amino acid having an optionally substituted alkyl group in a side chain or a secondary amino acid having an optionally substituted alkyl group;
X7 is an amino acid having an optionally substituted aromatic ring in a side chain;
X8 is an amino acid having an optionally substituted aromatic ring in a side chain;
X9 is any amino acid; and
X10 is any amino acid.

The "amino acid having an optionally substituted aromatic ring in a side chain" refers to an amino acid having an aromatic ring in the side chain and for example, having, as the aromatic ring, a phenyl group or indole ring, part (e.g., C) of which may be replaced by other molecule such as N. The aromatic ring may be heterocyclic. For example, the hydroxyl group of the side chain tyrosine may be replaced by other functional group. In one embodiment, the "amino acid having an optionally substituted aromatic ring in a side chain" is preferably an amino acid having a fused ring in the side chain. For example, the amino acid may have an indole ring or naphthalene structure, part (e.g., C) of which may be further replaced by other molecule such as N. The substituent is selected, if appropriate, and is not limited to an alkyl group, a cycloalkyl group, a hydroxyl group, or halogen, etc. Examples of the "amino acid having an optionally substituted aromatic ring in a side chain" include tyrosine, tryptophan, histidine, or phenylalanine. The amino acid may be a non-natural amino acid (e.g., Nal1, W6N, W7N, or W).

The "amino acid having an optionally substituted alkyl group in a side chain" may be an amino acid having a side chain alkyl group (e.g., lysine, alanine, proline, glycine, leucine). This amino acid is an amino acid having an optionally substituted or unsubstituted side chain. The alkyl group may be either linear or cyclic. The "secondary amino acid having an optionally substituted alkyl group" is a secondary amino acid and include any natural or non-natural amino acid with a cyclic or chain structure.

In one embodiment, the substituent is not particularly limited when the side chain of "amino acid having an optionally substituted alkyl group in a side chain" or "secondary amino acid having an optionally substituted alkyl group" is substituted. A functional group may be present at the terminus of the side chain alkyl group. Examples of the functional group attached to the terminus include, but are not limited to, an amino group or a benzyl group.

The above options for X1-X10 can be selected in any combination.

In one embodiment, X1 is an amino acid having an optionally substituted fused ring in a side chain. In one embodiment, X1 is Nal1, W6N, W7N, or W.

In one embodiment, X2 is V, K, KCOpipzaa, KCOmeglumine, or Q. In one embodiment, X2 is V, K, KCOpipzaa, or KCOmeglumine. In one embodiment, X2 is not E.

In one embodiment, X3 is Y, 4Py, A, E, F4G, Q,K, F3G, or 3Py. In one embodiment, X3 is Y or 4Py.

In one embodiment, X4 is an amino acid other than a substituted or unsubstituted aromatic or sulfur-containing amino acid. Examples of the "amino acid having an aromatic ring" include tyrosine, tryptophan, histidine, or phenylalanine. Examples of the "sulfur-containing amino acid" include methionine or cysteine. In one embodiment, X4 is D, A, Q, K, or E. In one embodiment, X4 is D.

In one embodiment, X5 is an amino acid other than a substituted or unsubstituted aromatic or sulfur-containing amino acid. In one embodiment, X5 is D, E, KCOpipzaa, KCOmeglumine, A, Q, or K. In one embodiment, X5 is D, E, KCOpipzaa, or KCOmeglumine.

In one embodiment, X6 is PeG, K, or MeG. In one embodiment, X6 is PeG. In one embodiment, X6 is not A, N, Q, S (D form), or P (D form).

In one embodiment, X7 is an amino acid having an optionally substituted fused ring in a side chain. In one embodiment, X7 is Nal1, W6N, or W. In one embodiment, X7 is Nal1. In one embodiment, X7 is not A, Q, K, or E.

In one embodiment, X8 is Y, 4Py, 3Py, or E. In one embodiment, X8 is Y or 4Py. In one embodiment, X8 is not A, Q, K, F3G, or F4G.

In one embodiment, X9 is an amino acid other than a substituted or unsubstituted aromatic or sulfur-containing amino acid. Examples of the "amino acid having an aromatic ring" include tyrosine, tryptophan, or phenylalanine. Examples of the "sulfur-containing amino acid" include methionine or cysteine. In one embodiment, X9 is H, A, Q, or E. In one embodiment, X9 is H.

In one embodiment, X10 is an amino acid other than a substituted or unsubstituted aromatic or sulfur-containing amino acid. Examples of the "amino acid having an aromatic ring" include tyrosine, tryptophan, histidine, or phenylalanine. Examples of the "sulfur-containing amino acid" include methionine or cysteine. In one embodiment, X10 is V, E, KCOpipzaa, KCOmeglumine, Q, K, or A. In one embodiment, X10 is V, E, KCOpipzaa, or KCOmeglumine.

The above options for X1-X10 in one of the above embodiments can be selected in any combination.

The above peptide is, in one embodiment, a cyclic peptide. The "cyclic peptide" means a peptide in which two amino acids are bonded and all or part of which is cyclic. The bonding is not limited to the type of bonding between two amino acids. Examples of the cyclic peptide include: a cyclic structure formed by an amide bond between a carboxy group of one amino acid and an amino group of other amino acid, a cyclic structure formed by a thioester bond between a carboxy group of one amino acid and a thiol group of other amino acid, a cyclic structure formed by a disulfide bond between a thiol group of one amino acid and a thiol group of other amino acid, or a cyclic structure formed by a lactam ring formation or macrocyclization reaction; or a cyclic peptide with a lasso peptide-like structure. Meanwhile, the two amino acids may be bonded therebetween by an amide bond. In this case, the amide bond is not limited to those formed by the bond between the carboxy group of one amino acid and the amino group of the other amino acid, but may be formed by an amide bond as a result of a synthetic reaction. The same applies to other types of bonding. Preferably, cyclization is formed by bonding a cysteine residue to a chloroacetylated amino acid as described below.

The cyclic peptide may have a part with a cyclic structure and may have a linear chain part.

Peptides generally have poor metabolic stability *in vivo,* and their large size makes it difficult for them to permeate the cell membrane. To address such issues, a peptide cyclization technique has been adopted. It has been suggested that the peptide cyclization increases protease resistance and thus increases their metabolic stability and restricts conformational changes, thereby increasing their rigidity and improving membrane permeability and affinity for a target protein.

In one embodiment, the peptide has a cyclic structure in which a chloroacetylated amino acid is bonded to a cysteine residue included in the peptide. In one embodiment, the peptide has a cyclic structure in which an N-terminal amino acid (the amino acid residue at position 1) is bonded to a cysteine residue included in the peptide. In one embodiment, the peptide has a cyclic structure in which an N-terminal amino acid (the amino acid residue at position 1) is bonded to a cysteine residue at position 16 included in the peptide. In one embodiment, the peptide has a cyclic structure in which a chloroacetylated N-terminal amino acid (the amino acid residue at position 1) is bonded to a cysteine residue at position 16 included in the peptide. The "chloroacetylation" may also be "halogen acetylation" using other halogen. Further, the "acetylation" may also be "acylation" with an acyl group other than the acetyl group.

As used herein, some amino acids may be modified for cyclization of a peptide. The present invention also encompasses amino acids that have been partially modified as such. For example, as described above, a chloroacetyl group may be added to the N-terminal amino acid, and may be bonded to a cysteine residue in the peptide to form a ring. Such various (natural/non-natural) amino acids containing a chloroacetyl group are also included in the present amino acids. In one embodiment, the peptide comprises or consists of an amino acid sequence set forth in any one of
SEQ ID NOs: 2 to 33 or
SEQ ID NOs: 2, 4-13, and 15-33 without a C-terminal glycine (G).

In one embodiment, the peptide is a cyclic peptide comprising or consisting of an amino acid sequences set forth in any one of
SEQ ID NOs: 2 to 33 or
SEQ ID NOs: 2, 4-13, and 15-33 without a C-terminal glycine (G).

In one embodiment, the peptide comprises or consists of an amino acid sequences set forth in any one of SEQ ID NOs: 34-69, or SEQ ID NOs: 34-69 without a C-terminal glycine (G).

In one embodiment, the peptide is a cyclic peptide comprising or consisting of an amino acid sequences set forth in any one of SEQ ID NOs: 34-69, or SEQ ID NOs: 34-69 without a C-terminal glycine (G).

The peptide may comprise an additional amino acid residue(s) in addition to an amino acid sequence set forth in any of SEQ ID NOs: 2-33, or SEQ ID NOs: 2, 4-13, and 15-33 without a C-terminal glycine (G). The peptide may comprise an additional amino acid residue(s) in addition to an amino acid sequence set forth in any one of SEQ ID NOs: 34-69, or SEQ ID NOs: 34-69 without a C-terminal glycine (G).

The additional amino acid residue(s) may be included in the peptide forming the cyclic structure, or the additional amino acid residue(s) may be added like a linker from the cyclic peptide. The peptide and the number of amide bonds (number and length of amino acids) in the peptide moiety are not limited. The total number of amino acid residues (i.e., the number of amino acid residues in the peptide forming the cyclic structure; if an additional amino acid residue(s) is added like a linker from the cyclic peptide, those amino acids are not included) is preferably within 22 residues. Preferably, the number of amino acids is 6 or more, 7 or more, 8 or more, 9 or more, 10 or more, 11 or more, or 12 or more, and preferably the number of amino acids is 20 or less, 19 or less, or 18 or less. More preferably, the number of amino acids is 15 or more and 18 or less and most preferably 16 or 17.

In addition, an additional linker may also be added to the cyclic peptide. Examples of the linker include the above-described amino acid linker (peptide linker), a chemical linker, a fatty acid linker, a nucleic acid linker, or a sugar chain linker. The linker may also be a complex of, for example, a chemical linker and a peptide linker. Examples of the chemical linker include a PEG (polyethyleneglycol) linker. For example, the PEG linker may consist of 1-24 ethylene glycol units. The linker may also be a fatty acid linker containing a divalent chemical moiety derived from a fatty acid. The amino acid (peptide) linker may be a linker containing at least one amino acid. It is possible to use, for example, a glycine-rich peptide such as a peptide having the sequence [Gly-Gly-Gly-Gly-Ser]ₙ (where n is 1, 2, 3, 4, 5, or 6) as described in U.S. Patent No. 7,271,149 or a serine-rich peptide linker described in U.S. Patent No. 5,525,491. A linker may be added without limitation to change the physical properties (e.g., solubility) of the peptide.

The linker may be added at any position. For example, it may be linked to Cys located on the C-terminal side, or to an amino acid contained in a cyclic peptide. Preferably, it is linked to Cys located on the C-terminal side, or to the side chain of an amino acid contained in a cyclic peptide. In one embodiment, the linker is contained at the C-terminus.

The linker may be added singly, or multiple linkers of the same or different types may be added. For example, Gly-Lys may be attached as an amino acid linker, and a PEG linker may be attached to the side chain terminus of the Lys.

Further, the peptide may form a multimer via, for example, a linker. In this case, the peptide may be a homomer consisting of peptides having the same sequence or a heteromer consisting of peptides having different sequences.

The glycine (G) at the C-terminus of SEQ ID NOs: 2, 4-13, and 15-33 is a linker. Similarly as described above for the peptide of SEQ ID NO: 2, the peptide may have a linker other than G in one embodiment. Alternatively, another linker may be further included in addition to G at the C-terminus.

In one embodiment, the peptide preferably has TGF-β binding activity and more preferably has TGF-β 1 binding activity or TGF-β2 binding activity.

In one embodiment, the peptide preferably has TGF-β 1 binding activity and TGF-β2 binding activity.

In one embodiment, the peptide preferably has TGF-β antagonist activity and more preferably has TGF-β 1 antagonist activity or TGF-β2 antagonist activity. Furthermore, in one embodiment, the peptide having TGF-β antagonist activity preferably has TGF-β1 binding activity. The term "having TGF-β1 binding activity" is not limited to the activity of binding only to TGF-β1, but may also have the activity of binding to TGF-β2.

In one embodiment, the peptide preferably has TGF-β 1 antagonist activity and TGF-β2 antagonist activity. In addition, the peptide having TGF-β1 antagonist activity and TGF-β2 antagonist activity preferably has TGF-β 1 binding activity and TGF-β2 binding activity.

TGF-β (transforming growth factor β) is a cytokine belonging to the TGF-β family. Three isoforms (TGF-β1, -β2, -β3) exist in mammals. In addition, the structurally similar TGF-β superfamily includes activin and BMPs (bone morphogenetic factors).

The "TGF-β" in an embodiment of the present invention is TGF-β1 and/or TGF-β2, and preferably TGF-β1. As used herein, the term "TGF-β" refers to TGF-β1 unless otherwise specified.

TGF-β binds to serine/threonine kinase-type receptors consisting of type I and type II receptor subunits and transduces a Smad phosphorylation-mediated signal. This induces a variety of reactions *in vivo.* For example, TGF-β is known to have multiple functions, such as regulating cell proliferation and differentiation, and promoting the production and deposition of extracellular matrix proteins (e.g., collagen, fibronectin) (Massague, 2012; Zhang *et al*., 2017).

Examples of TGF-β include, but are not limited to, TGF-β in mammals, such as primates (e.g., humans, chimpanzees), laboratory animals (e.g., rats, mice, rabbits), domestic animals (e.g., pigs, cows, horses, sheep, goats), and pet animals (e.g., dogs, cat), and preferably human TGF-β, and more preferably human TGF-β1 (NCBI GENE ID: 7040) or human TGF-β2 (NCBI GENE ID: 7042).

The "TGF-β1 binding activity" means the activity of binding specifically to TGF-β1, or the activity of binding specifically to both TGF-β1 and TGF-β2.

The state of binding the peptide of the present invention to TGF-β 1 can be expressed using, without limitation, affinity constant Ka, dissociation constant Kd (also denoted as KD), association rate constant kon, and dissociation rate constant koff as an indicator.

The affinity constant Ka and the dissociation constant Kd are indicators of the binding affinity, or association strength, between two molecules in equilibrium, and the dissociation constant Kd is obtained by inverting the affinity constant Ka. The smaller the value of the dissociation constant Kd, the stronger the binding. Meanwhile, the rate of association/dissociation reaction between two molecules in equilibrium is indicated by the association rate constant kon and the dissociation rate constant koff, which are calculated by the reaction kinetic analysis. Here, Kd = koff/kon. Thus, even in the case of equivalent dissociation constants Kd, there are two cases: one is a case of slow association but slow dissociation (both kon and koff values are small), and the other is a case of rapid association and rapid dissociation (both kon and koff values are large). Their respective binding retention states are quite different.

The state of binding the peptide to TGF-β can be expressed using, affinity constant Ka, dissociation constant Kd (also denoted as KD), association rate constant kon, and dissociation rate constant koff. They can be determined using any intermolecular interaction assay well-known to those skilled in the art.

The state of binding the peptide to TGF-β can be measured by known methods. For example, the state can be measured by surface plasmon resonance spectral analysis (SPR). Examples of the surface plasmon resonance spectral analysis that can be used include, but are not limited to, the BIACORE system (BIACORE, Inc.), which is a biosensor (bio-intermolecular interaction analyzer).

One indicator of the TGF-β binding activity of the peptide is the dissociation constant Kd. The lower the dissociation constant Kd, the higher the binding activity (affinity). According to surface plasmon resonance spectral analysis, the dissociation constant Kd for the binding between the peptide and, for example, human TGF-β is not limited but is 50 nM or less, 30 nM or less, 20 nM or less, 15 nM or less, 10 nM or less, 8 nM or less, 6 nM or less, or 5 nM or less. The lower limit of the dissociation constant Kd for the binding between the peptide and human TGF-β is not particularly limited. The dissociation constant Kd of the peptide is not limited but is 0.01 nM or more, 0.05 nM or more, 0.1 nM or more, 0.2 nM or more, 0.3 nM or more, 0.4 nM or more, or 0.5 nM or more. The dissociation constant Kd of the peptide is not limited but is 0.01-50 nM, preferably 0.05-30 nM, more preferably 0.2-15 nM, particularly preferably 0.3-10 nM, and most preferably 0.4-6 nM.

The binding the peptide to TGF-β can also be examined by ELISA (enzyme-linked immunosorbent assay), for example. ELISA may be performed, for example, by using TGF-β 1 beads and the peptide with an HA-tag sequence added to the C-terminus to test the binding activity.

The binding between the peptide and TGF-β is not limited but is preferably noncovalent.

In one embodiment, the peptide has the cyclic structure represented by the chemical formula of Examples 1-6.

The "TGF-β antagonist activity" refers to activity to inhibit or reduce the binding and action of TGF-β to TGF-β receptors. In the present invention, in particular, it is preferable to exert antagonist action by binding to TGF-β.

The "TGF-β antagonist activity" may be evaluated, for example, by an SBE reporter assay using the SBE reporter-HEK293 cell system (BP Bioscience). The inhibitory activity of the peptide can be calculated without limitation while the maximum signal induced by TGF-β is set to 0% inhibition and no stimulation is set to 100% inhibition. When the peptide is used at an appropriate concentration, for example, at the optimal concentration, the inhibitory activity is not limited but the inhibition is 5% or more, 10% or more, 20% or more, 30% or more, 40% or more, 50% or more, 60% or more, 70% or more, 80% or more, or 90% or more.

### 4. Production of Peptide

The peptide of the present invention can be produced by any known method for peptide production. Examples include the following:

a chemical synthesis method such as a liquid-phase method, a solid-phase method, a hybrid method in which liquid-phase and solid-phase methods are combined; and
a genetic recombinant method.

In the solid-phase method, for example, the hydroxyl group of a hydroxylated resin is esterified with the carboxy group of the first amino acid, α-amino group of which is protected by a protecting group (usually the C-terminal amino acid of the peptide of interest). The esterification catalyst used may be a known dehydration-condensation agent such as 1-mesitylene sulfonyl-3-nitro-1,2,4-triazole (MSNT), dicyclohexylcarbodiimide (DCC), or diisopropylcarbodiimide (DIC).

Next, the protecting group of the α-amino group of the first amino acid is eliminated and a second amino acid in which all functional groups other than the carboxy group of the main chain are protected is added, and the carboxyl group is activated, , and the first and second amino acids are bonded. Further, the α-amino group of the second amino acid is deprotected and a third amino acid in which all functional groups other than the carboxy group of the main chain are protected is added, and the carboxyl group is activated, and the second and third amino acids are bonded. This procedure is repeated until a peptide of the desired length is synthesized, and all the functional groups are then deprotected.

Examples of the resin for solid-phase synthesis include Merrifield resin, MBHA resin, Cl-Trt resin, SASRIN resin, Wang resin, Rink amide resin, HMFS resin, Amino-PEGA resin (Merck), or HMPA-PEGA resin (Merck). These resins may be washed with a solvent (e.g., dimethylformamide (DMF), 2-propanol, methylene chloride) before use.

Examples of the protecting group for α-amino group include a benzyloxycarbonyl (Cbz or Z) group, a *tert*-butoxycarbonyl (Boc) group, a fluorenylmethoxycarbonyl (Fmoc) group, a benzyl group, an allyl group, or an allyloxycarbonyl (Alloc) group. The Cbz group may be deprotected, for example, with hydrofluoric acid or by hydrogenation, the Boc group may be deprotected with trifluoroacetic acid (TFA), and the Fmoc group may be deprotected by treatment with piperidine or pyrrolidine.

The α-carboxy group may be protected using, for example, methyl ester, ethyl ester, allyl ester, benzyl ester, *tert*-butyl ester, or cyclohexyl ester.

The carboxy group may be activated using a condensation agent. Examples of the condensation agent include dicyclohexylcarbodiimide (DCC), diisopropylcarbodiimide (DIC), 1-ethyl-3-(3-dimethylaminopropyl)carbodiimide (EDC or WSC), (1H-benzotriazol-1-yloxy)tris(dimethylamino)phosphonium hexafluorophosphate (BOP), or 1-[bis(dimethylamino)methyl]-1H-benzotriazolium-3-oxide hexafluorophosphate (HBTU).

The peptide chain may be cleaved from the resin by treatment with an acid such as TFA or hydrogen fluoride (HF).

The peptide may be produced by genetic recombinant method (translation-synthesis system) while using a nucleic acid encoding the peptide described above. The nucleic acid encoding the peptide may be DNA or RNA.

The nucleic acid encoding the peptide may be prepared by a known method or an equivalent method. For example, the nucleic acid can be synthesized with an automatic synthesizer. The resulting DNA may contain a restriction enzyme recognition site(s) for vector insertion. Alternatively, a nucleotide sequence encoding an amino acid sequence may be incorporated, so that the resulting peptide chain can be cut out by enzyme or other means.

As mentioned above, in the case of fusion of the above peptide with, for instance, a membrane-permeable peptide, the above nucleic acid also includes a nucleic acid encoding the membrane-permeable peptide.

To inhibit degradation by host-derived proteases, a chimeric protein expression method, in which a target peptide is expressed as a chimeric peptide with other peptide, can also be used. In this case, it is possible to use, as the above nucleic acid, a nucleic acid encoding the target peptide and a peptide attached to the target peptide.

Then, an expression vector is prepared using the nucleic acid encoding the peptides. The nucleic acid can be inserted downstream of a promoter of the expression vector either as is or by digestion with restriction enzyme(s) or by adding a linker, etc. Examples of the vector include: an *E*. *coli*-derived plasmid (e.g., pBR322, pBR325, pUC12, pUC13, pUC18, pUC19, pUC118, pBluescript II); a *Bacillus subtilis*-derived plasmid (e.g., pUB 110, pTP5, pC1912, pTP4, pE194, pC194); a yeast-derived plasmid (e.g., pSH19, pSH15, YEp, YRp, YIp, YAC); a bacteriophage (e.g., e-phage, M13 phage); a virus (e.g., retrovirus, vaccinia virus, adenovirus, adeno-associated virus (AAV), cauliflower mosaic virus, tobacco mosaic virus, baculovirus); or a cosmid.

The promoter can be selected, if appropriate, according to the type of host. If the host is an animal cell, for example, an SV40 (simian virus 40)-derived promoter or a CMV (cytomegalovirus)-derived promoter can be used. If the host is *E. coli,* the trp promoter, T7 promoter, or lac promoter, for example, can be used.

The expression vector may have incorporated, for example, a DNA replication origin (ori), a selection marker (e.g., antibiotic resistance, nutrient requirement), an enhancer, a splicing signal, a poly-A addition signal, and/or a tag (e.g., FLAG, HA, GST, GFP)-encoding nucleic acid.

Next, an appropriate host cell is transformed with the expression vector. The host may be selected, if appropriate, in view of the relation to the vector. Examples of the host used include *E. coli, Bacillus subtilis,* a bacterium belonging to the genus *Bacillus,* yeast, an insect or insect cell, or an animal cell. Examples of the animal cell used include an HEK293T cell, a CHO cell, a COS cell, a myeloma cell, a HeLa cell, or a Vero cell. The transformation may be performed according to the type of host by a known method such as lipofection, a calcium phosphate method, electroporation, microinjection, particle gun, or other known methods. The transformants may be cultured according to a routine procedure to express a peptide of interest.

The peptide is purified from the transformant culture by collecting the cultured cells, by suspending the cells in an appropriate buffer solution, by, for instance, sonication or freeze-thawing to destroy the cells, and then by centrifugation or filtration to obtain a crude extract. If the peptide is secreted into the culture solution, the supernatant is collected.

Purification from the crude extract or culture supernatant can also be performed by a known method or an equivalent method (e.g., chlorination, dialysis, ultrafiltration, gel filtration, SDS-PAGE, ion exchange chromatography, affinity chromatography, reverse-phase high-performance liquid chromatography).

A known method or an equivalent method may be used to convert the resulting peptide in a free form to a salt, or the peptide in a salt form to a free form.

In one embodiment, the translation synthesis system may be a cell-free translation system. The cell-free translation system generally allows an expressed product to be obtained in a highly pure form without purification. The cell-free translation system contains, for example, ribosomal proteins, aminoacyl-tRNA synthetase (ARS), ribosomal RNA, amino acids, rRNA, GTP, ATP, translation initiation factor (IF), elongation factor (EF), termination factor (RF), and ribosome regeneration factor (RRF), and other factors necessary for translation. *E. coli* extract or wheat germ extract may be added to increase expression efficiency. In addition, rabbit red blood cell extract or insect cell extract may also be added.

Energy may be continuously supplied to the system containing them by using dialysis to produce, in a non-limiting manner, several hundred µg to several mg/mL of the protein. The system may also include RNA polymerase for transcription from the gene DNA. As a commercially available cell-free translation system that can be used, examples of the *E. coli*-derived system include Roche Diagnostics' RTS-100 (registered trademark), Gene Frontier's PURESYSTEM, or NEW ENGLAND Biolabs PURExpress In Vitro Protein Synthesis Kit; and examples of the system using wheat germ extract include those from ZOEGENE Corporation and CellFree Sciences.

In the cellular translation system, instead of aminoacyl-tRNA synthesized by natural aminoacyl-tRNA synthetase, an artificial aminoacyl-tRNA in which the desired amino acid or hydroxy acid is linked (acylated) to the tRNA may be used. Such aminoacyl-tRNA can be synthesized using an artificial ribozyme.

Examples of such a ribozyme include flexizyme (H. Murakami, H. Saito, and H. Suga, (2003), Chemistry & Biology, Vol.10, 655-662; and WO2007/066627 or others). The flexizyme is also known and called as the original Flexizyme (Fx) or a modified form such as dinitrobenzylflexizyme (dFx), enhanced Flexizyme (eFx), or aminoflexizyme (aFx).

By using the tRNA, generated by flexizyme, to which the desired amino acid or hydroxy acid is linked, the desired codon can be associated with the desired amino acid or hydroxy acid for translation. A special amino acid may be used as the desired amino acid. For example, the non-natural amino acid required for the cyclization described above can also be introduced into the peptide linked by this method.

The peptide may be chemically synthesized using various methods routinely used in the art. Examples of the method include stepwise solid-phase synthesis, semi-synthesis of peptide fragment via conformationally assisted re-ligation, or chemical ligation. The synthesis of the peptide is a chemical synthesis using various solid-phase techniques as described, for example, in K. J. Jensen, P. T. Shelton, S. L. Pedersen, Peptide Synthesis and Applications, 2nd Edition, Springer, 2013. The preferred strategy is based on a combination of a Fmoc group that temporarily protects the α-amino group and allows for selective removal by a base, and a protecting group that temporarily protects the side chain functional group and is stable under de-Fmoc conditions. The selection of such general peptide side chain is known in the above Peptide Synthesis and Applications, 2nd edition and G. B. Fields, R. L. Noble, Solid Phase Peptide Synthesis Utilizing 9-Fluorenylmethoxycarbonyl Amino Acids, Int. J. Peptide Protein Res. 35, 1990, 161-214, etc. Examples of the preferred peptide side chain protecting group include: a benzyl, *tert*-butyl, or trityl (Trt) group for the hydroxy group of serine or threonine; a 2-bromobenzyloxycarbonyl or *tert*-butyl group for the hydroxy group of tyrosine; a Boc, methyltetrazole thiol (Mtt), Alloc, or ivDde group for the amino group of the lysine side chain; a Trt or Boc group for the imidazole group of histidine; a 2,2,4,6,7-pentamethyldihydrobenzofuran-5-sulfonyl (Pbf) group for the guanidyl group of arginine; a *tert*-butyl, allyl, or 3-methylpentane (Mpe) group for the carboxy group of, for instance, glutamic acid or aspartic acid; a Trt group for the carboxamide group of glutamine or asparagine; or a Trt or monomethoxytrityl (Mmt) group for the thiol group of cysteine.

The peptide can be synthesized in a stepwise manner on the solid-phase resin described above. The α-amino protecting groups of the C-terminal amino acid used and all amino acids and the peptide used in the synthesis should be selectively removed in the synthetic process. Preferably, the above-described solid-phase resin is used to initiate the process. The C-terminal carboxy group of the peptide with the N-terminus appropriately protected with Fmoc, etc. or the C-terminal carboxy group of a Fmoc-protected amino acid is made into an activated ester with an appropriate reagent and then attached to the amino group on the solid-phase resin. Subsequent elongation of the peptide chain can be achieved by sequentially repeating the removal of the N-terminal protecting group (Fmoc group), followed by condensation with a protected amino acid derivative, according to the amino acid sequence of the peptide of interest. Note that the peptide of interest can be released at the final stage. For example, as the release conditions, TFA solution containing water/silylhydride/thiol as a scavenger in TFA, as described in, for instance, Teixeira, W. E. Benckhuijsen, P. E. de Koning, A. R. P. M. Valentijn, J. W. Drijfhout, Protein Pept. Lett., 2002, 9, 379-385, can be used for the release. A typical example is TFA/Water/TIS/DODT (volume ratio 92.5:2.5:2.5:2.5).

The peptide described herein may be synthesized using a single or multichannel peptide synthesizer, such as CEM's Liberty Blue synthesizer or Biotage's Syro I synthesizer or their successors.

The carboxy group may be activated using a condensation agent. Examples of the condensation agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris(dimethylamino)phosphonium hexafluorophosphate (BOP), or 1-[bis(dimethylamino)methyl]-1H-benzotriazolium-3-oxide hexafluorophosphate (HBTU).

The peptide can be cyclized according to a known method. Although not limited, for example, the peptide may be desined to contain two or more cysteine residues, so that a cyclic structure can be formed through a disulfide bond after translation. In addition, Goto's method (Y. Goto, et al., ACS Chem. Biol. 3 120-129 (2008)) may be used to synthesize a peptide with a chloroacetyl group at the N-terminus by genetic code reprogramming technique. In the peptide, a cysteine residue containing a sulfur molecule may be placed for cyclization. This causes the spontaneous nucleophilic attack of the mercapto group on the chloroacetyl group after translation, and the peptide is cyclized through a thioether bond. Other combination of amino acids that are bonded to form a ring may be placed, for cyclization, within the peptide by genetic code reprogramming technique (the present cyclization method: ClAc-Cys). Alternatively, the peptide may be cyclized by placing an L-2-amino adipic acid residue in the peptide and bonding it to the N-terminal main chain amino group. In this way, any known cyclization method can be used without restriction.

### 5. Complex

In one embodiment, the present invention relates to a complex. This complex is a complex comprising any of the above peptides, a linker linked to the peptide, and a substance attached to the linker. Since the peptide can bind to TGF-β, the complex can transfer the substance to TGF-β.

The form in which a substance (especially, an agent) is attached to a peptide is sometimes referred to as PDC (peptide drug conjugate).

The substance may be any substance desired by those skilled in the art, as long as it is desired to be delivered to TGF-β. Examples of the substance include, but are not limited to the followings:
compound: it includes a low-molecular-weight compound and medium-molecular-weight compound, e.g., a known low-molecular-weight agent;
peptide: it may be any peptide that binds to a target *in vivo* to exert some effect, e.g., any cyclic peptide different from the peptide of the present invention;
RI: it may be any compound that can be labeled with a radioisotope, such as a low-molecular-weight or medium-molecular-weight compound or an antibody that is labeled with a radioisotope, e.g., it includes a compound for PET examination;
protein: it may be any protein, such as an antibody or enzyme, that exhibits a useful function *in vivo,* e.g., it includes an enzyme used in enzyme replacement therapy;
nucleic acid: it may be any substance including a nucleotide sequence such as DNA and RNA, e.g., it includes a nucleic acid medicament;
molecule used for drug delivery systems (DDS): it may be a known molecule such as a liposome or micelle that is used for DDS, wherein the DDS molecule may further contain a compound such as an agent inside thereof.

The substance may be a complex formed by linking multiple substances listed above. The multiple substances linked may be of the same kind or of different kinds.

The form in which a substance (especially, an agent) is attached to a peptide is sometimes referred to as PDC (peptide drug conjugate). When mentioned herein, the "peptide" can also include the form of a substance-conjugated complex, unless otherwise specified.

### 6. Composition, etc.

The present invention also relates to a composition comprising the peptide of the present invention. In this section, the "peptide" may also include a complex in the "5. Complex".

The composition is not limited, and may be a medical composition, a diagnostic composition, or a research composition.

### Pharmaceutical Composition

The pharmaceutical composition may contain the peptide itself, and may contain a pharmaceutically acceptable salt of the peptide, or a solvate thereof. As used herein, the "peptide" may include a pharmaceutically acceptable salt thereof, or a solvate thereof, unless otherwise specified. The pharmaceutical composition preferably contains an effective amount of the peptide as an active ingredient.

The target disease for the "pharmaceutical composition" includes both diseases in which the above peptide itself is effective and diseases in which a peptide-modifying agent is effective. Examples of the target disease for the pharmaceutical composition include, but are not limited to, diseases related to the TGF-β signal transduction pathway, such as diseases related to cell proliferation (e.g., tumor, cancer, abnormal cell fibrosis). It is also useful as an active ingredient in each pharmaceutical composition for the treatment of renal dysfunction including glomerulosclerosis, liver cirrhosis, or myocardial infarction, etc. Alternatively, it may be targeted to diseases in which a peptide-modifying agent is effective.

The dosage form of the pharmaceutical composition herein is not particularly limited and may be administered orally or parenterally. Examples of the parenteral administration include an injection (e.g., an intramuscular, intravenous, or subcutaneous injection) or transdermal or transmucosal (nasal, oral, ocular, transpulmonary, transvaginal, rectal) administration.

The pharmaceutical composition may be modified in various ways in view of the easy-to-metabolize or easy-to-excrete characteristics of the polypeptide. For example, polyethylene glycol (PEG) or sugar chain may be added to the polypeptide to increase the residence time in blood and decrease the antigenicity. In addition, for example, a biodegradable polymer compound (e.g., polylactic acid glycol (PLGA)), porous hydroxyapatite, liposome, surface-modified liposome, an emulsion prepared with unsaturated fatty acid, nanoparticles, or nanospheres may be used as a sustained release base material, and the polypeptide can be encapsulated in them. For transdermal administration, a weak electric current can be applied to the skin surface for stratum corneum permeation (iontophoresis method).

The pharmaceutical composition may be made into a preparation by using the active ingredient as it is, or by adding a pharmaceutically acceptable carrier, excipient, and/or additive. Examples of the dosage form include liquid (e.g., an injection), dispersion, suspension, tablets, pills, powder, suppositories, pulvis, fine particles, granules, capsules, syrup, lozenges, inhalant, ointment, eye drops, nose drops, ear drops, or cataplasm. The preparation can be made by a routine procedure using, for example, an excipient, a binder, a disintegrant, a lubricant, a dissolving agent, a dissolution aid, a colorant, an odor correcting agent, a stabilizer, an emulsifier, an absorption enhancer, a surfactant, a pH modifier, a preservative, and/or an antioxidant as appropriate.

Examples of the component used in the preparation include, but are not limited to, purified water, saline, phosphate buffer, dextrose, glycerol, a pharmaceutically acceptable organic solvent (e.g., ethanol), animal or vegetable oil, lactose, mannitol, glucose, sorbitol, crystalline cellulose, hydroxypropyl cellulose, starch, cornstarch, silicic anhydride, magnesium aluminum silicate, collagen, polyvinyl alcohol, polyvinyl pyrrolidone, a carboxy vinyl polymer, sodium carboxymethylcellulose, sodium polyacrylate, sodium alginate, watersoluble dextran, sodium carboxymethyl starch, pectin, methylcellulose, ethylcellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohol, stearyl alcohol, stearic acid, or human serum albumin.

In view of the fact that peptides are generally difficult to be absorbed transmucosally, the pharmaceutical composition may contain an absorption enhancer for improving absorption of a poorly absorbable drug. Examples of such an absorption enhancer include: a surfactant (e.g., a polyoxyethylene lauryl ether compound, sodium lauryl sulfate, saponin); a salt of bile acid (e.g., glycolic acid, deoxycholic acid, taurocholic acid); a chelator (e.g., EDTA, salicylic acid); a fatty acid compound (e.g., caproic acid, capric acid, lauric acid, oleic acid, linoleic acid, a mixed micelle); or an enamine derivative, an N-acyl collagen peptide, N-acylamino acid, a cyclodextrin compound, a chitosan compound, or a nitric oxide donor.

If the pharmaceutical composition is a pill(s) or a tablet(s), it may be coated with a sugar coating or a gastric-soluble or enteric-soluble substance.

If the pharmaceutical composition is an injection, it may contain distilled water for injection, physiological saline, propylene glycol, polyethylene glycol, vegetable oil, and/or an alcohol compound. In addition, it is possible to add a wetting agent, an emulsifier, a dispersant, a stabilizer, a dissolving agent, a dissolution aid, and/or a preservative.

The pharmaceutical composition may be targeted not only to humans but also to non-human mammals or birds. Examples of the non-human mammals include non-human primates (e.g., monkeys, chimpanzees, gorillas), domestic animals (e.g., pigs, cows, horses, sheep), or dogs, cats, rats, mice, guinea pigs, or rabbits.

In particular, the dose when administered to a human, varies depending on the symptoms, age, sex, body weight, and sensitivity of the patient as well as the administration method, the administration interval, the type of active ingredient, and the type of preparation. The dose is not limited and the dose of, for example, from 30 µg to 100 g, from 100 µg to 500 mg, or from 100 µg to 100 mg can be administered in one or several separate portions. For injection, 1 µg/kg to 3000 µg/kg or 3 µg/kg to 1000 µg/kg may be administered once or in several separate doses depending on the patient's body weight.

The present invention relates to a method of preventing or treating a disease by administering the peptide of the present invention.

The present invention relates to use of the peptide of the present invention for prevention or treatment of a disease.

The present invention relates to use of the peptide of the present invention for the manufacture of a pharmaceutical composition for prevention or treatment of a disease.

The present invention relates to the peptide of the present invention for use in a method of preventing or treating a disease.

### Diagnostic or Research Composition

The present invention also relates to a diagnostic composition comprising the peptide of the present invention.

The peptide preferably has TGF-β binding activity and more preferably has TGF-β1 binding activity. The term "having TGF-β1 binding activity" is as described in detail in "3. Peptide (B)". Since binding to TGF-β, the peptide of the present invention can be used as a diagnostic agent to detect TGF-β. As a diagnostic agent, the peptide of the present invention may be used to detect the amount of TGF-β. When used as a detection agent, the peptide of the present invention may be detectably labeled. The peptide of the present invention may be labeled by a label and may form a complex with TGF-β. This enables the detection or quantification of TGF-β via the label.

The type of label is not limited. Examples include, but are not limited to, a fluorescent material (e.g., fluorescein isothiocyanate, rhodamine, dansyl chloride, phycoerythrin, tetramethylrhodamine isothiocyanate, a near infrared fluorescent material), a chemiluminescent dye,. a radioactive substance (e.g., a radioisotope (e.g., ¹²⁵I, ¹³¹I, ³⁵S, ³H), or a chelate complex of radioisotope metal ion (e.g. a chelate complex of DOTA or desferrioxamine), a fluorescent protein (e.g., biotin, GFP (green fluorescent protein)), a luminescent protein, or an enzyme (e.g., peroxidase, alkaline phosphatase). Examples of the preferred label include a fluorescein derivative (e.g., fluorescein, FITC), a rhodamine derivative (e.g., rhodamine, tetramethylrhodamine), or a fluorescent dye (e.g., Texas Red).

The disease that can be diagnosed with the diagnostic composition is not limited. Examples include diseases related to the TGF-β signal transduction pathway, such as diseases related to cell proliferation (e.g., tumor, cancer, abnormal cell fibrosis). It is also useful as a diagnostic composition for renal dysfunction including glomerulosclerosis, liver cirrhosis, or myocardial infarction, etc.

The present invention relates to a method of diagnosing a disease by using the peptide of the present invention. The "diagnosis method" include *in vivo* or *in vitro* diagnosis methods. Preferred is an *in vitro* diagnosis method. The present invention also relates to a method of detecting a disease by using the peptide of the present invention. The disease can be detected by, for instance, laboratory technicians or researchers other than physicians, at research institutes (including educational institutions such as universities), companies, or others. In one embodiment, the method of detecting a disease does not include medical treatment.

The present invention relates to use of the peptide of the present invention for diagnosis or detection of a disease.

The present invention relates to use of the peptide of the present invention for the manufacture of a composition for diagnosis or detection of a disease.

The present invention relates to the peptide of the present invention for use in a method of diagnosing or detecting a disease.

The present invention also relates to a research composition comprising the peptide of the present invention. However, the research composition of the present invention excludes any composition used as an additive to a medium for culturing an organoid. The "research composition" include those used by, for instance, researchers, technicians, students, or doctors in research institutes (including educational institutions such as universities), companies, hospitals, or others.

The research composition can be used, for example, for purification or detection of TGF-β or detection of a disease.

In one embodiment, the peptide of the present invention has TGF-β antagonist activity and may thus be used as an additive to a medium for culturing cells or an organ(s).

It can also be used as an additive to a protective solution to protect an organ for transplantation. The medium is not limited and may be used to culture an organ or fragment thereof (e.g., a liver or brain stem) and may be used to culture cells with pluripotency, such as stem cells. Furthermore, it can be used in cell culture for cell therapy in regenerative medicine, for example. The "research composition" of the present invention includes a composition used for such a medium.

However, the research composition of the present invention excludes any composition used as an additive to a medium for culturing an organoid. The "organoid" is derived from human stem cells and cultured three-dimensionally due to its self-aggregation, self-renewal, and differentiation potentials. Thus, the organoid is an *in vitro* culture formed by self-assembly. The "organoids" are also considered to be "miniature versions of organs".

The peptide has TGF-β, especially binding activity against TGF-β 1 (affinity) and has particularly high TGF-β binding activity. Thus, it is possible to selectively isolate TGF-β from human serum, for example. Therefore, the above peptide can be used in the method of purifying TGF-β. The method of purifying TGF-β includes binding the peptide or immobilized peptide to TGF-β, and recovering TGF-β by releasing the bound TGF-β. Further, the above peptide can be used in the method of detecting TGF-β. The method of detecting TGF-β includes binding the peptide or immobilized peptide to TGF-β in a sample, and detecting the bound TGF-β. Here, the detection includes either qualitative or quantitative analysis.

The carrier for immobilizing the peptide is not limited herein. Examples include a glass, metal, or resin-made microtiter plate, substrate, or bead, or a nitrocellulose, nylon, or PVDF membrane.

The present invention includes a method of purifying or detecting TGF-β by using the peptide of the present invention.

The present invention also relates to use of the peptide of the present invention for purification or detection of TGF-β.

The present invention also relates to a diagnostic or detection kit comprising the peptide of the present invention. The diagnostic or detection kit includes a reagent and a tool (including, but is not limited to, any or all of the peptide of the present invention, an antibody, a solid-phase carrier, a buffer solution, an enzyme reaction-stopping solution, and/or a microplate reader) necessary for the above detection.

The present invention relates to a diagnostic or detection tester comprising the peptide of the present invention.

### EXAMPLES

Hereinafter, the present invention will be described in detail based on Examples. The present invention, however, is not limited to these Examples. A those skilled in the art may easily make modifications and changes to the present invention based on the description herein, and they are included in the technical scope of the present invention.

### Chemical Synthesis

All raw materials, building blocks, reagents, acids, bases, solid-phase resins, and solvents used in the chemical syntheses in the following Examples are either commercially available products or can be synthesized using organic chemistry by those skilled in the art. Note that a protecting group-containing amino acids are commercially available products unless otherwise noted.

Peptide chain elongation on a solid-phase resin was performed using each resin described in the respective Examples as a starting material under common peptide coupling reaction conditions and Fmoc removal reaction conditions. The reactions were performed using CEM's Liberty Blue or Biotage's Syro I, an automated peptide synthesizer, according to the manufacturer's instruction.

As examples, some of the common amino acids used are listed below, and each side chain protecting group is indicated in parentheses.
Fmoc-Trp(Boc)-OH;
Fmoc-Gly-OH;
Fmoc-Asp(OMpe)-OH;
Fmoc-Phe-OH;
Fmoc-His(Boc)-OH;
Fmoc-Tyr(tBu)-OH;
Fmoc-Val-OH;
Fmoc-Cys(Trt)-OH;
Fmoc-Asn(Trt)-OH.

Each obtained crude peptide was purified by elution using reverse-phase preparative HPLC on a Waters AutoPurification System-SQD2 single quadruple mass spectrometer while monitoring m/z ions derived from the target peptide. It was checked as to whether the mass spectrum obtained in the ESI-positive scan mode and the mass spectrum including multi-valent ions calculated from the molecular formula of the target product agreed within the error range of the mass spectrometer used. Note that the purification conditions, including the column(s) used, are shown in the respective Examples.

The structure of each chemically synthesized peptide was determined by ESI-MS(+) in mass spectrometry to confirm the molecular weight calculated by considering the amino acids used according to the target sequence and the building blocks used as necessary. Note that the term "ESI-MS(+)" indicates electrospray ionization mass spectrometry performed in positive ion mode. The detected masses are reported in "m/z" units. Note that when the molecular weight was approximately larger than 1000, the compound was frequently detected as a divalent or trivalent ion.

### Example 1: Synthesis of TGFb1_001 (SEQ ID NO: 2)

In this Example, TGFb1_001 (SEQ ID NO: 2) having the following structure was synthesized.

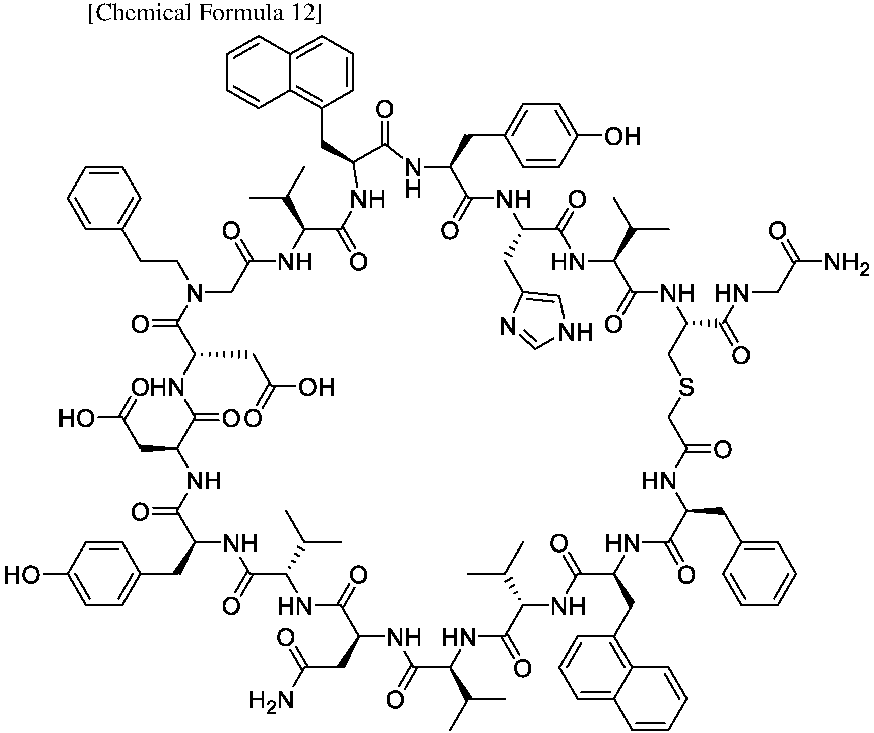

The target peptide was synthesized by the general chemical synthesis method described above while using Fmoc-Sieber amide resin (0.47 mmol/g, 0.213 g; Watanabe Chemical Industries, LTD.) and removing the Fmoc group at first. At that time, CEM's Liberty Blue HT was used as a solid-phase synthesizer for synthesis according to the manufacturer's instruction.

For the introduction of each residue, Fmoc-AA/DIPCI/Oxyma pure (5.25 equivalents/10 equivalents/5 equivalents) was used per equivalent of resin, and the reaction was carried out once for 3 min at 90°C in DMF. Provided that the reaction was carried out twice for residue 9 at 90°C for 10 min. The reaction was carried out once for residues 14 and 16 at 50°C for 15 min.

Meanwhile, the basic conditions for Fmoc removal were a reaction with a 20% piperidine-containing DMF solution at 75°C for 3 min. Provided that the Fmoc removal reaction was carried out twice for residues 1, 2, 3, 4, 5, 6, 7, and 8 by using 20% piperidine-containing DMF solution at 25°C for 5 min.

A chloroacetyl group was introduced as follows: the Fmoc group of the α-amino group was removed from the the Fmoc-protected peptide obtained in the previous step which was immobilized to solid-phase resin, by the method described above; and a DMF solution containing 0.2 M chloroacetic acid (about 5 equivalents), a DMF solution containing 0.5 M HATU (about 5 equivalents), and a DMF solution containing 1 M DIEA (about 10 equivalents), per equivalent of resin, were added to the solid-phase resin and the mixture was shaken for 30 min at room temperature.

The deprotection of the side chain and the cleavage from the solid-phase resin were performed as follows: first, the resin obtained after the chloroacetyl group introduction step was washed with DMF 5 times and methylene chloride 3 times; after that, the resin was dried under reduced pressure; then, a reaction agent cocktail-A (5 mL of a mixture of TFA/H₂O/TIS/DODT at a volume ratio of 92.5:2.5:2.5:2.5) was added to a reaction vessel containing the solid-phase resin, which was shaken at room temperature for 60 min.

The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a precipitate occurred. The mixture was centrifuged (at 9000 rpm for 1 min), and the supernatant was then decanted. The resulting solid was washed again 3 times with 15 mL of diethyl ether cooled to 0°C and then dried under reduced pressure. The solid obtained was used in the next cyclization reaction.

For the peptide cyclization reaction, the peptide was dissolved in DMSO to a final concentration of 5 mM based on the number of moles of solid-phase resin, and 10 equivalents of triethylamine was added. The mixture was then shaken at room temperature for about 5 hours. The resulting reaction solution was concentrated under reduced pressure by using a Genevac's EZ-2 (less than 40°C; reduced pressure duration: 14 hours).

The resulting crude product was purified using the following conditions. Column: Waters Xbridge (registered trademark) C18 5 µm 30 × 150 mm; mobile phase: A = 0.1% TFA (in H₂O), B = 0.1% TFA (in MeCN); temperature: 40°C; gradient (%B): 13-38% over 3 min, then 38-43% over 8 min, then 43-60% over 1 min; flow rate: 45 mL/min.

The purity of the target product was 96.0% as calculated from the area ratio of LC/MS (UV wavelength 225 nm) chromatogram under the analytical conditions.

Analytical conditions: retention time = 15.01 min; column: Kinetex EVO C18 2.6 µm 2.1 × 150 mm, 100 Å; mobile phase: A = 0.025% TFA (in H₂O), B = 0.025% TFA (in MeCN); temperature: 60°C; gradient (%B conc): 20-60% over 20 min, then 60-95% over 1 min, then 95-95% over 5 min; flow rate: 0.25 mL/min.

ESI-MS(+): observed m/z=1112.53 (M+2H)²⁺

### Example 2: Synthesis of TGFb1_012 (SEQ ID NO: 13)

In this Example, TGFb1_012 (SEQ ID NO: 13) having the following structure was synthesized.

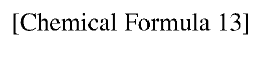

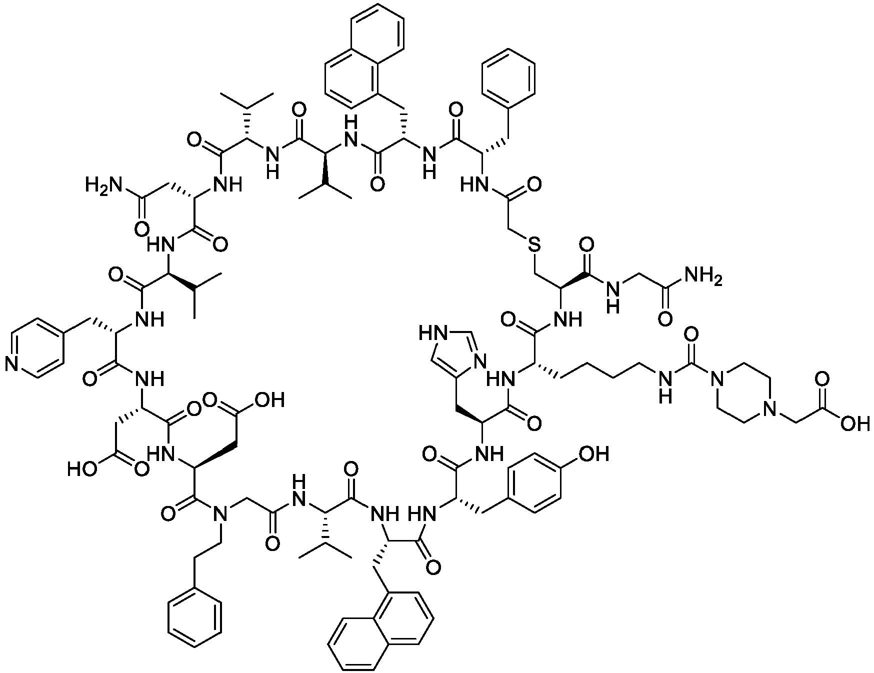

The target peptide was synthesized by the general chemical synthesis method described above while using Fmoc-Sieber amide resin (0.65 mmol/g, 0.115 g; Watanabe Chemical Industries, LTD.) and removing the Fmoc group at first. At that time, Biotage's Syro I was used as a solid-phase synthesizer for synthesis according to the manufacturer's instruction.

For the introduction of each residue, Fmoc-AA/HATU/DIPEA (4.2 equivalents/4 equivalents/8 equivalents) was used per equivalent of resin, and the reaction was carried out twice for 20 min at 75°C in DMF. Provided that the reaction was carried out once for residues 11, 12, and 13 at 75°C for 20 min. The reaction was carried out once for residue 14 at room temperature for 60 min. The reaction was carried out once for residue 15 at 75°C for 20 min. The reaction was carried out once for residue 17 at 75°C for 10 min. The reaction was carried out once for residue 16 at room temperature for 20 min.

Meanwhile, the basic conditions for Fmoc removal were a reaction with a 20% piperidine-containing DMF solution at room temperature for 5 min and for 15 min.

A chloroacetyl group was introduced as follows: the Fmoc group of the α-amino group was removed from the the Fmoc-protected peptide obtained in the previous step which was immobilized to solid-phase resin by the method described above;; and a DMF solution containing 0.2 M chloroacetic acid (about 5 equivalents), a DMF solution containing 0.2 M DIPCI (about 5 equivalents), and a DMF solution containing 0.2 M HOSu (about 5 equivalents), per equivalent of resin, were added to the solid-phase resin and the mixture was shaken for 90 min at room temperature.

The deprotection of the side chain and the cleavage from the solid-phase resin were performed as follows: first, the resin obtained after the chloroacetyl group introduction step was washed with DMF 5 times and methylene chloride 3 times; after that, the resin was dried under reduced pressure; then, a reaction agent cocktail-A (3 mL of a mixture of TFA/H₂O/TIS/DODT at a volume ratio of 92.5:2.5:2.5:2.5) was added to a reaction vessel containing the solid-phase resin, which was shaken at room temperature for 60 min.

The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropyl ether cooled to 0°C, a precipitate occurred. The mixture was centrifuged (at 9000 rpm for 3 min), and the supernatant was then decanted. The resulting solid was washed again 3 times with 10 mL of diethyl ether cooled to 0°C and then dried under reduced pressure. The solid obtained was used in the next cyclization reaction.

For the peptide cyclization reaction, the peptide was dissolved in DMSO/H₂O (9/1) mixed solution to a final concentration of 2.5 mM based on the number of moles of solid-phase resin, and 10 equivalents of triethylamine was added. The mixture was then shaken at room temperature for about 18.5 hours. The reaction solution was admixed with 20 equivalents of acetic acid. The resulting reaction solution was concentrated under reduced pressure by using a Genevac's EZ-2 (less than 40°C; reduced pressure duration: 16 hours).

The resulting crude product was purified using the following conditions. Column: Waters Xbridge (registered trademark) C18 5 µm 19 × 150 mm; mobile phase: A = 0.1% TFA (in H₂O), B = 0.1% TFA (in MeCN); temperature: 60°C; gradient (%B): 5-30% over 3 min, then 30-35% over 8 min, then 35-60% over 1 min; flow rate: 17 mL/min.

The purity of the target product was 90.5% as calculated from the area ratio of LC/MS (UV wavelength 225 nm) chromatogram under the analytical conditions.

Analytical conditions: retention time = 3.62 min; column: Kinetex EVO C18 2.6 µm 2.1 × 150 mm, 100 Å; mobile phase: A = 0.025% TFA (in H₂O), B = 0.025% TFA (in MeCN); temperature: 60°C; gradient (%B conc): 20-60% over 7.15 min, then 60-95% over 0.3 min, then 95-95% over 1.55 min; flow rate: 0.5 mL/min.

ESI-MS(+): observed m/z=1204.64 (M+2H)²⁺

### Example 3: Synthesis of TGFb1_031 (SEQ ID NO: 32)

In this Example, TGFb1_031 (SEQ ID NO: 32) having the following structure was synthesized.

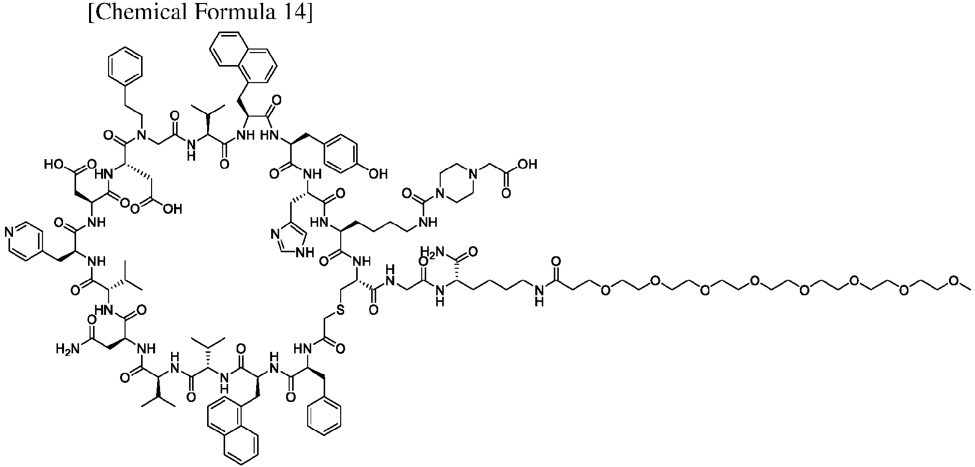

The target peptide was synthesized by the general chemical synthesis method described above while using Fmoc-Sieber amide resin (0.48 mmol/g, 0.521 g; Watanabe Chemical Industries, LTD.) and removing the Fmoc group at first. At that time, CEM's Liberty Blue HT was used as a solid-phase synthesizer for synthesis according to the manufacturer's instruction.

For the introduction of each residue, Fmoc-AA/DIPCI/Oxyma pure (4.2 equivalents/8 equivalents/4 equivalents) was used per equivalent of resin, and the reaction was carried out once for 3 min at 90°C in DMF. Provided that the reaction was carried out once for residues 3, 4, 6, 7, 11, and 15 at 75°C for 20 min. The reaction was carried out twice for residue 9 at 90°C for 3 min. The reaction was carried out twice for residue 10 at 75°C for 20 min. The reaction was carried out once for residue 14 at 50°C for 15 min.

Meanwhile, the basic conditions for Fmoc removal were a reaction with a 20% piperidine-containing DMF solution at 75°C for 3 min. Provided that the Fmoc removal reaction was carried out twice for residues 1, 2, 3, 4, 5, 6, 7, 8, 11, 12, 13, 15, 17, and 18 by using 20% piperidine-containing DMF solution at 25°C for 5 min.

A chloroacetyl group was introduced as follows: the Fmoc group of the α-amino group was removed from the the Fmoc-protected peptide obtained in the previous step which was immobilized to solid-phase resin by the method described above; and a DMF solution containing 0.1 M chloroacetic acid (about 5 equivalents), a DMF solution containing 0.1 M HATU (about 5 equivalents), and a DMF solution containing 0.2 M DIEA (about 10 equivalents), per equivalent of resin, were added to the solid-phase resin and the mixture was shaken for 30 min at room temperature.

The deprotection of the side chain and the cleavage from the solid-phase resin were performed as follows: first, the resin obtained after the chloroacetyl group introduction step was washed with DMF 5 times and methylene chloride 3 times; after that, the resin was dried under reduced pressure; then, a reaction agent cocktail-A (5 mL of a mixture of TFA/H₂O/TIS/DODT at a volume ratio of 92.5:2.5:2.5:2.5) was added to a reaction vessel containing the solid-phase resin, which was shaken at room temperature for 60 min.

The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a precipitate occurred. The mixture was centrifuged (at 9000 rpm for 2 min), and the supernatant was then decanted. The resulting solid was washed again 3 times with 15 mL of diethyl ether cooled to 0°C and then dried under reduced pressure. The solid obtained was used in the next cyclization reaction.

For the peptide cyclization reaction, the peptide was dissolved in DMSO to a final concentration of 5 mM based on the number of moles of solid-phase resin, and 10 equivalents of triethylamine was added. The mixture was then shaken at room temperature for about 16 hours. The resulting reaction solution was concentrated under reduced pressure by using a Thermo Scientific Savant Explorer SpeedVac (less than 40°C; reduced pressure duration: 3 hours).

The resulting crude product was purified using the following conditions. Column: Waters Xbridge (registered trademark) C18 5 µm 50 × 150 mm; mobile phase: A = 0.1% TFA (in H₂O), B = 0.1% TFA (in MeCN); temperature: 40°C; gradient (%B): 5-28% over 3 min, then 28-33% over 8 min, then 33-60% over 1 min; flow rate: 120 mL/min.

The purity of the target product was 99.2% as calculated from the area ratio of LC/MS (UV wavelength 225 nm) chromatogram under the analytical conditions. The peptide obtained here is designated as peptide A.

Analytical conditions: retention time = 1.20 min; column: CSH Ph-hexyl 1.7 µm 2.1 × 30 mm; mobile phase: A = 0.025% TFA (in H₂O), B = 0.025% TFA (in MeCN); temperature: 60°C; gradient (%B conc): 5-95% over 1.14 min, then 95-95% over 0.86 min; flow rate: 0.72 mL/min.

ESI-MS(+): observed m/z=1269.3 (M+4H)⁴⁺

The peptide A (16.5 µmol) obtained above was dissolved in DMSO/H₂O (9/1) mixed solution to a final peptide concentration of 25 mM, and then admixed with 1.2 equivalents of MePEG8c, 1.1 equivalents of HATU, and 3 equivalents of DIPEA. The mixture was shaken at room temperature for about 1 hour.

The resulting crude product was purified using the following conditions. Column: Waters Xbridge (registered trademark) C18 5 µm 19 × 150 mm; mobile phase: A = 0.1% TFA (in H₂O), B = 0.1% TFA (in MeCN); temperature: 50°C; gradient (%B): 7-32% over 3 min, then 32-37% over 8 min, then 37-60% over 1 min; flow rate: 17 mL/min.

The purity of the target product was 98.2% as calculated from the area ratio of LC/MS (UV wavelength 225 nm) chromatogram under the analytical conditions.

Analytical conditions: retention time = 4.09 min; column: Kinetex EVO C18 2.6 µm 2.1 × 150 mm, 100 Å; mobile phase: A = 0.025% TFA (in H₂O), B = 0.025% TFA (in MeCN); temperature: 60°C; gradient (%B conc): 20-60% over 7.15 min, then 60-95% over 0.3 min, then 95-95% over 1.55 min; flow rate: 0.5 mL/min.

ESI-MS(+): observed m/z=977.70 (M+3H)³⁺

### Example 4: Synthesis of TGFb1_032 (SEQ ID NO: 33)

In this Example, TGFb1_032 (SEQ ID NO: 33) having the following structure was synthesized.

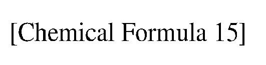

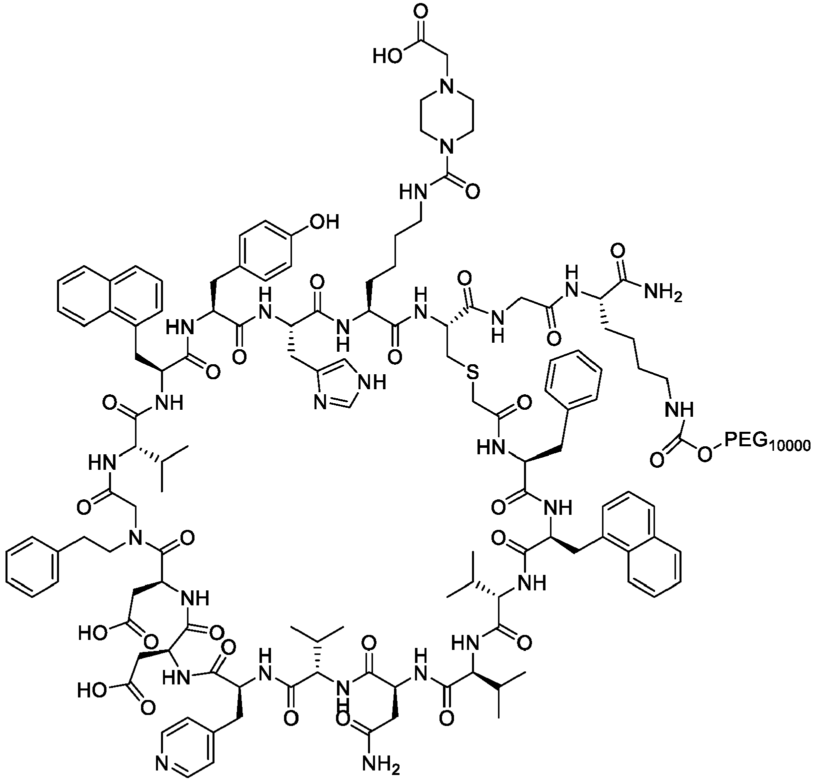

The peptide A (4.5 µmol) obtained through the synthesis of TGFb1_031 (SEQ ID NO: 32) was dissolved in acetonitrile/H₂O (1/1) mixed solution to a final peptide concentration of 25 mM, and then admixed with 0.67 equivalents of NHS-OCOmPEG10000 and 5 equivalents of DIPEA. The mixture was shaken at room temperature for about 1 hour.

The resulting crude product was purified using the following conditions. Column: Waters Xbridge (registered trademark) C18 5 µm 30 × 150 mm; mobile phase: A = 0.1% TFA (in H₂O), B = 0.1% TFA (in MeCN); temperature: 50°C; gradient (%B): 35-35% over 0.5 min, then 35-60% over 11.5 min; flow rate: 45 mL/min.

The purity of the target product was 99.3% as calculated from the area ratio of LC/MS (UV wavelength 225 nm) chromatogram under the analytical conditions.

Analytical conditions: retention time = 3.16 min; column: Kinetex EVO C18 2.6 µm 2.1 × 150 mm, 100 Å; mobile phase: A = 0.025% TFA (in H₂O), B = 0.025% TFA (in MeCN); temperature: 60°C; gradient (%B conc): 40-80% over 7.15 min, then 80-95% over 0.3 min, then 95-95% over 1.55 min; flow rate: 0.5 mL/min.

ESI-MS(+): observed m/z=1331.06 (median)

### Example 5: Synthesis of TGFb1_024 (SEQ ID NO: 25)

In this Example, TGFb1_024 (SEQ ID NO: 25) having the following structure was synthesized.

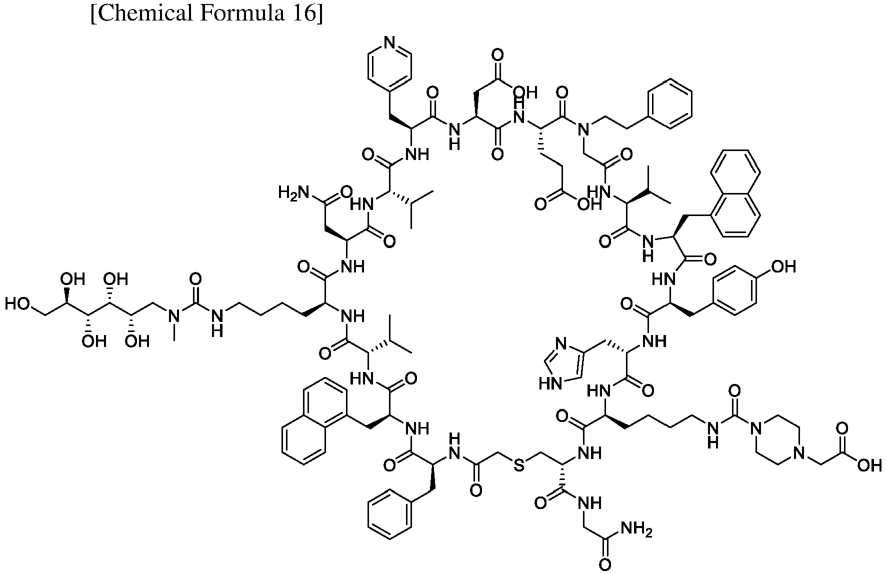

The target peptide was synthesized by the general chemical synthesis method described above while using Fmoc-Sieber amide resin (0.48 mmol/g, 0.156 g; Watanabe Chemical Industries, LTD.) and removing the Fmoc group at first. At that time, Biotage's Syro I was used as a solid-phase synthesizer for synthesis according to the manufacturer's instruction.

For the introduction of each residue, Fmoc-AA/HATU/DIPEA (4.2 equivalents/4 equivalents/8 equivalents) was used per equivalent of resin, and the reaction was carried out twice for 20 min at 75°C in DMF. Provided that the reaction was carried out twice for residues 4 and 9 at 75°C for 30 min. The reaction was carried out twice for residue 14 at room temperature for 30 min. The reaction was carried out once for residue 15 at 75°C for 20 min. The reaction was carried out once for residue 16 at room temperature for 20 min. The reaction was carried out once for residue 17 at 75°C for 10 min.

Meanwhile, the basic conditions for Fmoc removal were a reaction with a 20% piperidine-containing DMF solution at room temperature for 5 min and for 15 min.

Introduction of the chloroacetyl group, deprotection of the side chain, and cleavage from the solid-phase resin, as well as precipitation of the peptide, were performed according to the method for the synthesis of TGFb1_012 (SEQ ID NO: 13).

For the peptide cyclization reaction, the peptide was dissolved in acetonitrile/H₂O (1/1) mixed solution to a final concentration of 2.5 mM based on the number of moles of solid-phase resin, and 10 equivalents of triethylamine was added. The mixture was then shaken at room temperature for about 3 hours. The reaction solution was admixed with 20 equivalents of acetic acid. The resulting reaction solution was concentrated under reduced pressure by using a Genevac's EZ-2 (less than 40°C; reduced pressure duration: 16 hours).

The resulting crude product was purified using the following conditions. Column: Waters Xbridge (registered trademark) C18 5 µm 50 × 150 mm; mobile phase: A = 0.1% TFA (in H₂O), B = 0.1% TFA (in MeCN); temperature: 40°C; gradient (%B): 5-26% over 3 min, then 26-31% over 8 min, then 31-60% over 1 min; flow rate: 17 mL/min.

The purity of the target product was 79.9% as calculated from the area ratio of LC/MS (UV wavelength 225 nm) chromatogram under the analytical conditions.

Analytical conditions: retention time = 3.01 min; column: Kinetex EVO C18 2.6 µm 2.1 × 150 mm, 100 Å; mobile phase: A = 0.025% TFA (in H₂O), B = 0.025% TFA (in MeCN); temperature: 60°C; gradient (%B conc): 20-60% over 7.15 min, then 60-95% over 0.3 min, then 95-95% over 1.55 min; flow rate: 0.5 mL/min.

ESI-MS(+): observed m/z=1336.96 (M+2H)²⁺

### Example 6: Synthesis of TGFb1_068 (SEQ ID NO: 69)

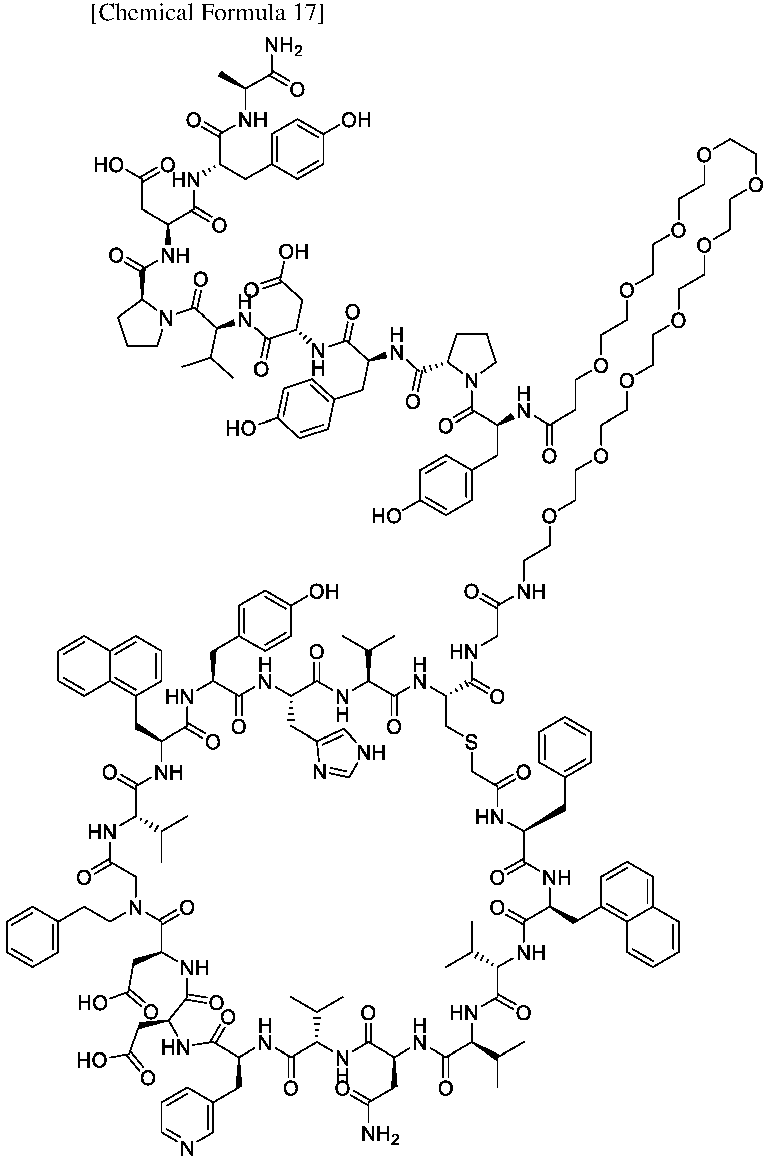

The target peptide was synthesized by the general method described above while using HA-tag resin (0.65 mmol/g, 0.004 mg, 2.5 µmol scale) and removing the Fmoc group at first. At that time, Biotage's Syro II was used as a solid-phase synthesizer for synthesis according to the manufacturer's instruction.

Fmoc-AA/HATU/DIPEA (4.2 equivalents/3.9 equivalents/8.4 equivalents) was used per equivalent of solid-phase resin, and the reaction was carried out twice for 20 min at 75°C in DMF. Provided that the reaction was carried out twice for residues 5, 7, 8, 9, 10, 12, 13, and 17 at 75°C for 10 min. The reaction was carried out twice for residues 14 and 16 at 50°C for 30 min. The reaction was carried out once for residue 18 at room temperature for 60 min. Meanwhile, the basic conditions for Fmoc removal were a reaction with a 20% piperidine-containing DMF solution at room temperature for 5 min and for 15 min.

A chloroacetyl group was introduced as follows: the Fmoc group of the α-group was removed from the the Fmoc-protected peptide obtained in the previous step which was immobilized to solid-phase resin by the method described above;; and a DMF solution containing 0.3 M chloroacetic acid (4.2 equivalents), a DMF solution containing 0.28 M HATU (3.9 equivalents), and a DMF solution containing 1.05 M DIPEA (8.4 equivalents), per equivalent of resin, were added to the solid-phase resin and the mixture was shaken for 30 min at room temperature, which was repeated twice.

The deprotection of the side chain and the cleavage from the solid-phase resin were performed as follows: first, the resin obtained after the chloroacetyl group introduction step was washed with DMF 5 times and methylene chloride 3 times; after that, the resin was dried under reduced pressure; then, a reaction agent cocktail-A (a mixture of TFA/H₂O/TIS/DODT at a volume ratio of 92.5:2.5:2.5:2.5) was added to a reaction vessel containing the solid-phase resin, which was shaken at room temperature for 60 min.

The reaction solution was collected by filtration through a frit. The solid-phase resin remaining in the reaction vessel was shaken again with the cocktail for cleavage, and the solution component was collected from the frit and mixed with the above filtrate. When the filtrate was added to diisopropyl ether (1.5 mL) cooled to 0°C, a precipitate occurred. The mixture was centrifuged (at 8500 rpm for 30 seconds), and the supernatant was then decanted and removed. The resulting solid was washed again twice with diethyl ether/hexane (1:1; 1 mL) cooled to 0°C and then dried under reduced pressure. The solid obtained was used in the next cyclization reaction.

For the peptide cyclization reaction, the peptide was dissolved in isopropyl alcohol/H₂O/DMSO (1:1:18) to a final concentration of 25 mM (2.5 µtmol/0.1 mL) based on the number of moles of solid-phase resin, and 20 equivalents of triethylamine was added. The mixture was then shaken at room temperature for 63 hours. The resulting reaction solution was admixed with 40 equivalents of acetic acid.

The resulting reaction solution was subjected to solid-phase extraction using a Gilson column (column: Gilson ASPEC C18 500 mg 3 mL).
(i) The column was washed with extractant A (0.1% TFA in 95% MeCN/water; 3 mL).
(ii) The column was equilibrated with extractant B (0.1% TFA in 5% MeCN/water; 3 mL).
(iii) 0.1 mL of the above reaction solution was loaded onto the column.
(iv) The column was washed with extractant B (4 mL).
(v) Extracted with extractant A (4 mL). The resulting extract was concentrated under reduced pressure by using EZ-2 Elite.

The purity of one of the major peaks of the target product was 21.8% as calculated from the area ratio of LC/MS (UV wavelength 220 nm) chromatogram under the following analytical conditions.

Analytical conditions: retention time = 1.74 min; column: Kinetex EVO C18 1.7 µm 2.1 × 50 mm, 100 Å; mobile phase: A = 0.025% TFA (in H₂O), B = 0.025% TFA (in MeCN); temperature: 60°C; gradient (%B conc): 5-95% over 2.10 min, then 95-95% over 0.75 min; flow rate: 0.6 mL/min.

ESI-MS(+): observed m/z=1903.5 (M+2H)²⁺

### Example 7: Synthesis of Various Peptides

In this Example, like Examples 1 to 6, various cyclic peptides were chemically synthesized. Tables 1 and 4 show the sequence of each synthesized peptide. In Table 1, SEQ ID NOs: 2, 4-13, and 15-33 each have a glycine (G) linker added to the C-terminus of a cyclic peptide having an amino acid sequence with a cyclized structure. SEQ ID NO: 32 has an additional K (MePEG8c) added to the end of the G linker. Then, SEQ ID NO: 33 has an additional K (OCOmPEG10000) added to the end of the G linker. Note that SEQ ID NO: 3 has the same amino acid sequence as SEQ ID NO: 2, but without the C-terminal glycine (G) linker addition. Note that SEQ ID NO: 14 has the same amino acid sequence as SEQ ID NO: 13, but without the C-terminal glycine (G) linker addition.

Peptides consisting of the amino acid sequences set forth in SEQ ID NOs: 34-69 also each have a G attached as a linker to the C-terminus of the cyclic peptide having a cyclized structure. Note that in Table 4, an HA-tag is attached to the G end of the C-terminal linker of the peptide consisting of the amino acid sequence set forth in SEQ ID NOs: 34-69. This is the amino acid sequence required to evaluate the binding of a TGF-β inhibitory peptide to TGF-β 1 by ELISA and does not affect the activity.

The synthesized peptides were analyzed under the analytical conditions described in Examples 1 to 6, and their structures were checked by ESI-MS(+) in the mass spectrometry. The resulting observed ESI-MS(+) values and the valence in such cases are shown in Tables 1 and 4.

### Example 8: Test for Evaluating Intermolecular Interaction between TGF-β and Peptide by Surface Plasmon Resonance (SPR)

In this Example, (1) the activity of binding to TGF-β for various peptides consisting of the amino acid sequences set forth in SEQ ID NOs: 2-33 synthesized in Examples 1 to 5 and 7 was evaluated. (2) The activity and selectivity of binding of the peptide consisting of the amino acid sequence set forth in SEQ ID NO: 13 synthesized in Example 2 against two isoforms of TGF-β (TGF-β1 and β2) were checked. For this purpose, the intermolecular interaction of each peptide against TGF-β was tested by surface plasmon resonance (SPR) in the method described below. Specific test methods are described below.

### [SPR measurement]

A CM3 sensor chip (Global Life Science Technologies Japan Co., Ltd.) was inserted into a BiacoreT200 (Global Life Science Technologies Japan Co., Ltd.). Three priming runs were performed using running buffer: HBS-EP+ (Global Life Science Technologies Japan Co., Ltd.). The system was then equilibrated at a flow rate of 30 µL/min.

Subsequently, 50 µL of 60 mM EDC solution (Global Life Science Technologies Japan Co., Ltd.) and 50 µL of 650 mM NHS solution (Global Life Science Technologies Japan Co., Ltd.) were mixed. Next, the reaction was carried out at a flow rate of 10 µL/min for 420 seconds. Then, 150 µL of 0.2 µM TGF-β solution diluted in 10 mM acetic acid solution (pH 5.0) was prepared and reacted at a flow rate of 10 µL/min for 420 seconds to immobilize TGF-β on the CM3 sensor chip. TGF-β was human-derived TGF-β1 or β2 (recombinant human TGF-β1, 2) and was obtained from R&D systems. After immobilization, capping was performed by reacting with 1.0 M ethanolamine aqueous solution (Global Life Science Technologies Japan Co., Ltd.) at a flow rate of 10 µL/min for 420 seconds.

A liquid prepared by dissolving each peptide at 10 mM in DMSO solution was diluted with running buffer to prepare a peptide-dissolved solution at a final concentration of 10 µM. Subsequently, 50 nM, 25 nM, 12.5 nM, 5 nM, and 2.5 nM peptide solutions were prepared. Using the above samples, the kinetics of each peptide against TGF-β was obtained by SPR measurement.

The kinetics evaluation model was Single Cycle Kinetics, and curve fitting was performed using Biacore T200 Evaluation Software Version 3.0 (Global Life Science Technologies Japan Co., Ltd.). The binding of various peptides to TGF-β was evaluated by performing least-squares curve fitting on the obtained sensorgram to calculate the KD value.
(1) KD values for peptides consisting of the amino acid sequences set forth in SEQ ID NOs: 2-33 are shown in Tables 2 and 3. As shown in Tables 2 and 3, the peptides of SEQ ID NOs: 2-33 were demonstrated to have ability of binding to TGF-β1. The peptide set forth in SEQ ID NO: 3 or 14 has the same amino sequence that forms a cyclic peptide structure as SEQ ID NO: 2 or 13, respectively. The only difference is the presence or absence of glycine added at the linker site. The ability of binding the peptide to TGF-β1 was 0.46 nM for KD of the peptide of SEQ ID NO: 3 and 0.82 nM for the peptide of SEQ ID NO: 14. These values were almost the same as the KDs of SEQ ID NOs: 2 and 13, to which glycine was added. This suggests that the peptide can bind to TGF-β1 even when a linker is added to the peptide of the present invention.
(2) The KD values of the peptide consisting of the amino acid sequence set forth in SEQ ID NO: 13 against two isoforms of TGF-β (TGF-β1 and β2) were 0.86 nM for TGF-β1 and 2.0 nM for TGF-β2, as described in Table 2. The above results have demonstrated that the peptide of the present invention binds specifically to TGF-β 1 and 2.

### Example 9: To Evaluate TGF-β Inhibitory Activity

In this Example, the TGF-β1 inhibitory activity of the peptide of the present invention consisting of the amino acid sequence set forth in SEQ ID NOs: 2-33 synthesized in Examples 1 to 5 and 7 was evaluated by an SBE reporter assay using the SBE reporter-HEK293 cell system (BP Bioscience) as follows.

In addition, the selectivity of each peptide for TGF-β isoforms (TGF-β1 and β2) is checked. For this purpose, the inhibitory activity against TGF-β 1 and TGF-β2 was evaluated using the peptide consisting of the amino acid sequence set forth in SEQ ID NO: 13 synthesized in Example 2.

SBE reporter-HEK293 cells were cultured in E-MEM (Wako) containing 10% FBS (Sigma). The cells were detached using Accutase (Innovative Cell Technologies), suspended in E-MEM containing 0.5% FBS, seeded onto a 96-well plate(s) at a concentration of 45,000 cells per well, and cultured overnight. The next day, the peptide was added and allowed to stand for 20 min, and recombinant human TGF-β1 (R&D systems) or recombinant human TGF-β2 (R&D systems) was then added at 0.13 nM and cultured for 18 hours.

Thereafter, luciferase assay ONE-Glo reagent was added to each well and the plate was shaken for 5 min. A SpectraMax Paradigm multimode microplate reader (Molecular Devices) was then used for signal detection. The obtained signals were analyzed by GraphPad Prism. The inhibitory activity of the peptide was calculated while the maximum signal induced by TGF-β was set to 0% inhibition and no stimulation was set to 100% inhibition.

Tables 2 and 3 show the results of evaluating the TGF-β1 inhibitory activity of the peptide consisting of the amino acid sequence set forth in SEQ ID NOs: 2-33 synthesized in Examples 1 to 5 and 7. Tables 2 and 3 have demonstrated that various peptides synthesized have the TGF-β1 antagonist activity. The peptide of SEQ ID NO: 3 or 14 has the same amino sequence that forms the cyclic peptide structure as SEQ ID NO: 2 or 13, respectively, as described above, and only difference is the presence or absence of glycine in the linker moiety. The binding activity is also not changed as indicated by Example 8. Therefore, the SBE reporter results are omitted.

In addition, Fig. 1 show the results of evaluating the selectivity for TGF-β isoforms (TGF-β1 and β2). Fig. 1 has demonstrated the inhibitory activity, against TGF-β1 or TGF-β2, of the peptide consisting of the amino acid sequence set forth in SEQ ID NO: 13 synthesized in Example 2. This indicates that the peptide consisting of the amino acid sequence set forth in SEQ ID NO: 13 of the present invention inhibits not only TGF-β 1 but also TGF-β2.

### Example 10: To evaluate binding between peptide and TGF-β by ELISA

In this Example, ELISA was used to evaluate the binding between TGF-β1 and each of various peptides consisting of the amino acid sequences set forth in SEQ ID NOs: 34-69 synthesized in Examples 6 and 7. An HA-tag was attached to the G end of the C-terminal linker of the peptide consisting of the amino acid sequence set forth in SEQ ID NOs: 34-69. As used herein, the "HA-tag" means the amino acid sequence of PEG10c-Y-P-Y-D-V-P-D-Y-A (SEQ ID NO: 70). This is the amino acid sequence required to evaluate the binding of a TGF-β inhibitory peptide to TGF-β 1 by ELISA and does not affect the TGF-β1 binding activity.

The binding activity of each peptide to TGF-β 1 was examined by ELISA using the peptide consisting of the amino acid sequence set forth in SEQ ID NOs: 34-69 having an HA-tag attached and TGF-β1 beads.

### <To prepare TGF-β1 Beads>

Recombinant human TGF-β 1 (R&D Systems) was immobilized on Dynabead M-270 Carboxylic Acid (Thermo fisher scientific) to prepare TGF-β1 beads. The immobilization reaction was performed using the Amine Coupling kit (DOJINDO LABORATORIES).

### <ELISA>

TGF-β1 beads and each peptide having the HA-tag sequence attached at the C-terminus were reacted in PBS (PBS-T) containing 0.1% Tween 20 (NACALAI TESQUE, INC.) for 1 hour and then washed by PBS-T. To detect the peptide bound to the TGF-β1 beads, an anti-HA-tag mAb-HRP-DirecT (MBL) and the TGF-β1 beads were reacted in PBS-T containing 1% BSA for 30 min. After washing with PBST, a SureBlue TMB Microwell Peroxidase Substrate (Kirkegaard & Perry Laboratories) was added for coloration for 10 min. TMB stop solution (Kirkegaard & Perry Laboratories) was then added to stop the coloration reaction. An EnSpire (Perkin Elmer) was used to measure absorbance.

Note that the binding activity was expressed as a relative value when the ability of binding the peptide TGFb1_061 (SEQ ID NO: 62) to TGF-β1 was set to 100%. Table 4 shows the results. Table 4 has demonstrated the ability of the peptide consisting of the amino acid sequence set forth in SEQ ID NOs: 34-69 to bind to TGF-β1.

Table 1: Peptide sequences that exhibited antagonist activity and MS values (SEQ ID NOs: 2-33)

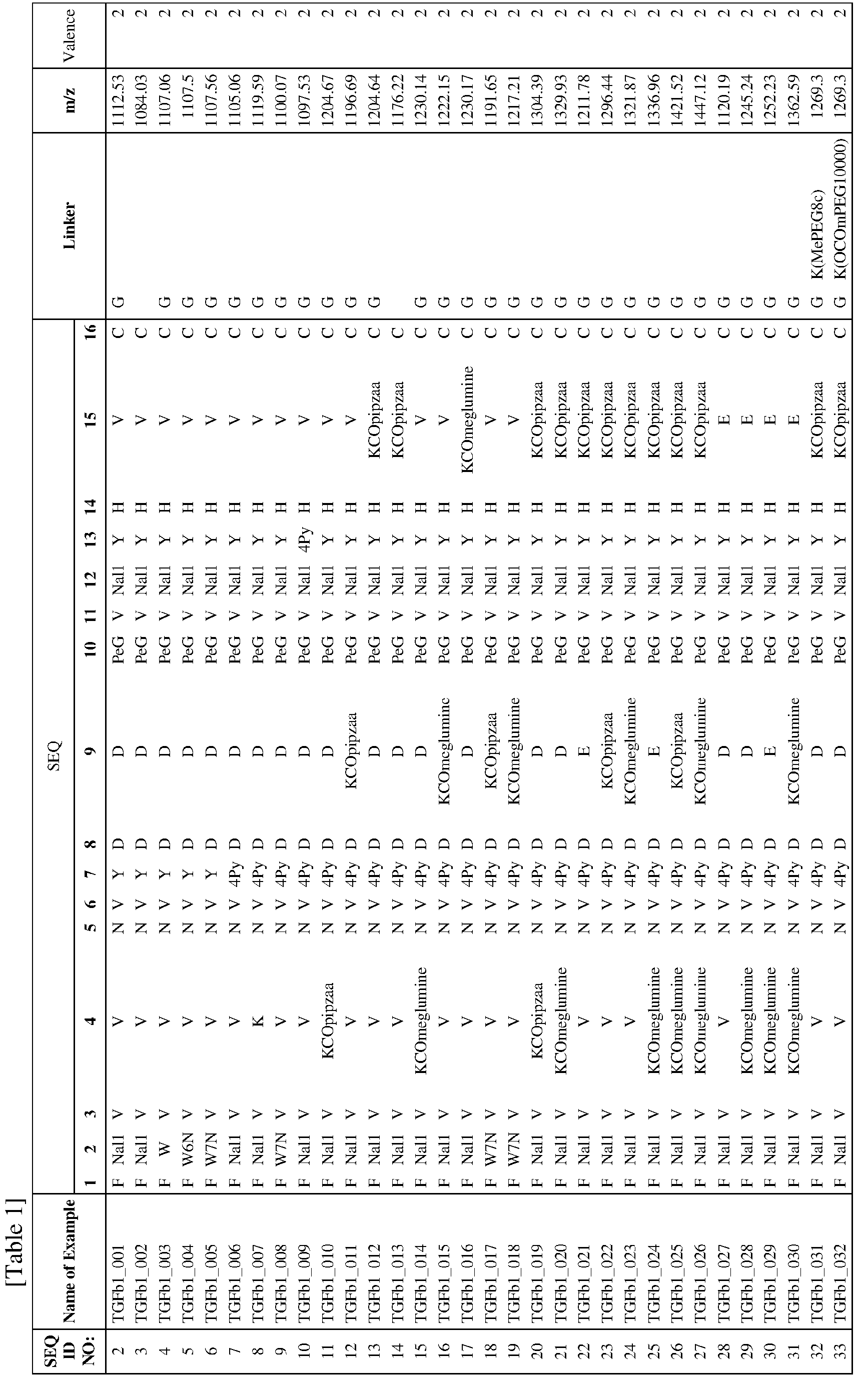

The m/z for SEQ ID NO: 32 or 33 in Table 1 is the value before the structure MePEG8c or OCOmPEG10000 was added to the linker K(MePEG8c) or K(OCOmPEG10000), respectively.

Table 2: Results of measuring SPR and antagonist activity of peptide sequence set forth in SEQ ID NOs: 2-19

**[Table 2]**

| **SEQ ID NO:** | **Name of Example** | **KD (nM)** | **SBE reporter %inhibition** | | | | |
|---|---|---|---|---|---|---|---|
| | | | (nM) | 0.075 | 0.75 | 7.5 | 75.0 |
| 2 | TGFb1_001 | 0.55 | | 19.7 | 69.7 | 98.1 | 100.6 |
| 4 | TGFb1_003 | 0.72 | | 1.6 | 65.2 | 97.3 | 98.8 |
| 5 | TGFb1_004 | 1.49 | | 0.0 | 0.9 | 70.1 | 94.9 |
| 6 | TGFb1_005 | 1.15 | | 0.4 | 47.0 | 90.8 | 98.9 |
| 7 | TGFb1_006 | 1.03 | | 2.8 | 69.3 | 97.1 | 99.7 |
| 8 | TGFb1_007 | 2.70 | | 0.1 | 37.5 | 83.5 | 96.7 |
| 9 | TGFb1_008 | 4.64 | | 3.8 | 56.8 | 94.6 | 98.4 |
| 10 | TGFb1_009 | 0.58 | | 2.7 | 53.0 | 94.8 | 99.6 |
| 11 | TGFb1_010 | 0.90 | | 2.0 | 63.8 | 97.9 | 99.9 |
| 12 | TGFb1_011 | 0.62 | | 6.1 | 76.3 | 97.9 | 100.0 |
| 13 | TGFb1_012 | 0.86 | | 10.1 | 65.2 | 96.0 | 99.5 |
| 15 | TGFb1_014 | 0.66 | | 0.0 | 64.8 | 97.2 | 98.8 |
| 16 | TGFb1_015 | 0.59 | | 0.0 | 76.9 | 98.7 | 98.4 |
| 17 | TGFb1_016 | 0.97 | | 6.1 | 61.0 | 97.6 | 100.2 |
| 18 | TGFb1_017 | 1.07 | | 21.7 | 60.2 | 97.2 | 100.0 |
| 19 | TGFb1_018 | 1.42 | | 22.2 | 61.0 | 97.6 | 99.9 |

Table 3: Results of measuring SPR and antagonist activity of peptide sequence set forth in SEQ ID NOs: 20-33

**[Table 3]**

| **SEQ ID NO:** | **Name of Example** | **KD (nM)** | **SBE reporter %inhibition** | | | | | | |
|---|---|---|---|---|---|---|---|---|---|
| | | | (nM) | 0.062 | 0.185 | 0.56 | 1.7 | 5.0 | 15.0 |
| 13 | TGFb1_012 | 0.86 | | 4.18 | 19.75 | 48.37 | 80.93 | 94.42 | 98.68 |
| 20 | TGFb1_019 | 1.41 | | 5.21 | 11.33 | 30.18 | 61.64 | 87.75 | 96.36 |
| 21 | TGFb1_020 | 1.26 | | 8.50 | 15.76 | 36.31 | 70.43 | 89.30 | 96.94 |
| 22 | TGFb1_021 | 1.37 | | 10.20 | 26.85 | 58.89 | 87.77 | 97.12 | 98.85 |
| 23 | TGFb1_022 | 0.91 | | 6.49 | 20.84 | 53.76 | 85.23 | 97.14 | 99.48 |
| 24 | TGFb1_023 | 1.04 | | 8.58 | 24.45 | 49.71 | 82.35 | 95.98 | 99.52 |
| 25 | TGFb1_024 | 1.04 | | 5.79 | 16.65 | 45.05 | 80.24 | 95.55 | 98.87 |
| 26 | TGFb1_025 | 0.83 | | 4.81 | 18.38 | 43.34 | 77.51 | 94.70 | 99.42 |
| 27 | TGFb1_026 | 1.03 | | 6.85 | 17.80 | 41.38 | 74.29 | 93.39 | 98.04 |
| 28 | TGFb1_027 | 1.11 | | 2.53 | 9.14 | 28.41 | 59.38 | 84.91 | 95.51 |
| 29 | TGFb1_028 | 1.31 | | 0.94 | 6.17 | 20.36 | 47.63 | 73.91 | 91.72 |
| 30 | TGFb1_029 | 0.94 | | 11.97 | 16.91 | 32.43 | 63.66 | 85.78 | 96.75 |
| 31 | TGFb1_030 | 1.13 | | 10.88 | 14.30 | 29.95 | 52.55 | 80.82 | 95.25 |
| 32 | TGFb1_031 | 0.91 | | 9.29 | 20.14 | 51.88 | 84.94 | 96.73 | 99.09 |
| 33 | TGFb1_032 | 3.08 | | 2.67 | 7.18 | 1.11 | 4.77 | 53.99 | 91.32 |

The concentrations (nM) under the section of SBE reporter in Tables 2 and 3 indicate the actual peptide concentrations used in the measurements.

Table 4: Peptide sequences that exhibited the activity of binding to TGF-β, although antagonist activity was not measured, MS values, and results of measuring the activity of binding to TGF-β (SEQ ID NOs: 34-69)

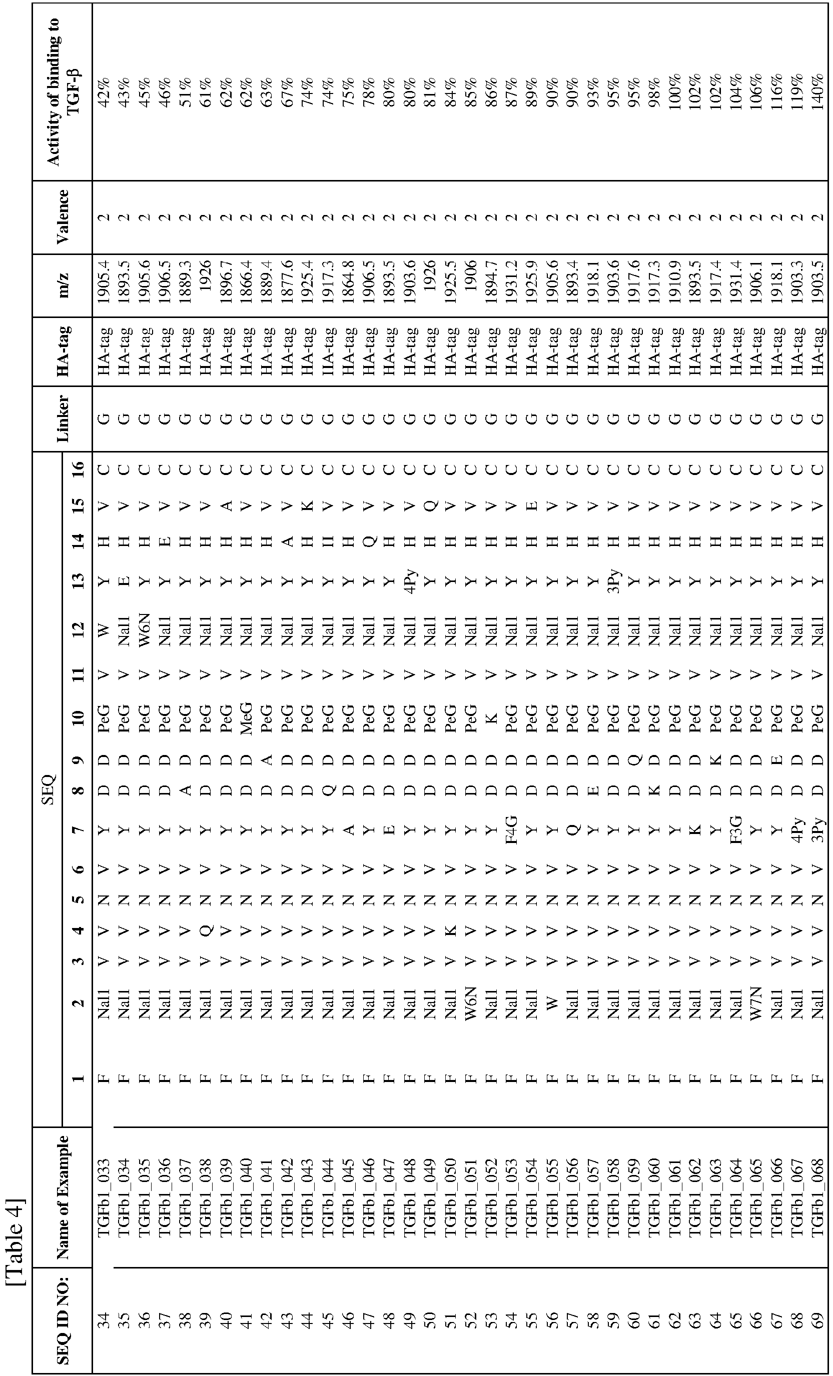

The activity of binding to TGF-β in Table 4 was expressed as a relative value when the ability of the peptide TGFb1_061 (SEQ ID NO: 62) to bind to TGF-β1 was set to 100%. Reference Example: Synthesis of Novel Non-Natural Amino Acid

This Reference Example shows the synthesis of a non-natural amino acid contained in the peptide of the present invention.

### Synthesis of KCOmeglumine

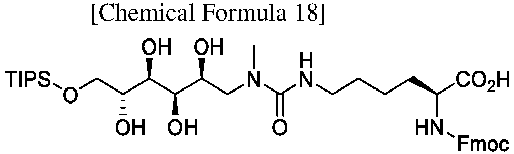

To a solution containing N2-{[(9H-fluoren-9-yl)methoxy]carbonyl}-L-lysine-2-propen-1-yl ester hydrochloride (8.00 g, 17.98 mmol) in dichloromethane (80 mL) was added triphosgene (2.67 g, 8.99 mmol) under ice-cold conditions. DIEA (10.1 mL, 57.5 mmol) was added dropwise and the mixture was then stirred under ice-cold conditions for 30 min. Dioxane (80 mL) was added to the reaction solution, followed by 0.5 mol/L sodium bicarbonate aqueous solution (80 mL) containing meglumine (14.0 g, 71.9 mmol). The reaction solution was stirred overnight under ice-cold conditions. The reaction solution was diluted with ethyl acetate. The organic layer was washed with water, 0.5 mol/L citric acid aqueous solution, saturated sodium bicarbonate aqueous solution, and saturated saline, and dried over sodium sulfate. The solution was filtered and the filtrate was concentrated under reduced pressure. The resulting residue was purified by silica gel column chromatography (dichloromethane/methanol = 100/0 to 60/40) to afford the desired product (8.70 g, 13.82 mmol) as a white amorphous product.

The resulting product (8.70 g, 13.82 mmol) was dissolved in dichloromethane (100 mL), and admixed with imidazole (2.35 g, 34.5 mmol) and triisopropylchlorosilane (3.33 g, 17.27 mmol) under ice-cold conditions. The mixture was then stirred at room temperature for 5 days. The reaction solution was diluted with ethyl acetate and washed three times with water. The extracted layer was dried over sodium sulfate and filtered, and the filtrate was concentrated under reduced pressure.

The resulting residue was purified by silica gel column chromatography (ethyl acetate/hexane = 0/100 to 60/40, then dichloromethane/methanol = 100/0 to 90/10) to afford the desired product (9.30 g, 11.83 mmol) as a white amorphous product.

The resulting product (10.4 g, 13.23 mmol) was dissolved in dichloromethane (50 mL), and admixed with phenylsilane (3.3 mL, 26.5 mmol) and tetrakis(triphenylphosphine)palladium(0) (0.38 g, 0.331 mmol). The mixture was then stirred at room temperature for 2 hours. The reaction solution was concentrated and then purified by silica gel column chromatography (ethyl acetate/hexane = 0/100 to 60/40, then dichloromethane/methanol = 100/0 to 90/10) to afford the titled substance (7.7 g, 10.3 mmol).

ESI-MS(+): observed m/z=746.6 (M+H)⁺

### INDUSTRIAL APPLICABILITY

The peptide of the present invention has TGF-β binding activity and preferably TGF-β antagonist activity, and is applicable for a pharmaceutical composition (e.g., for cancer, and liver diseases), a diagnostic composition, and a research composition.

## Claims

1. A peptide comprising the following amino acid sequence:
F-Nal1-V-V-N-V-Y-D-D-PeG-V-Nal1-Y-H-V-C-G (SEQ ID NO: 2);
or an amino acid sequence having a substitution, addition, deletion, or insertion in 1 to 11 amino acid residues selected from the group consisting of amino acid residues at positions 2, 4, 7, 8, 9, 10, 12, 13, 14, 15, and 17 in the above amino acid sequence.

2. A peptide comprising the following amino acid sequence:
F-X1-V-X2-N-V-X3-X4-X5-X6-V-X7-X8-X9-X10-C (SEQ ID NO: 1) wherein
X1 is an amino acid having an optionally substituted aromatic ring in a side chain;
X2 is any amino acid;
X3 is any amino acid;
X4 is any amino acid;
X5 is any amino acid;
X6 is an amino acid having an optionally substituted alkyl group in a side chain or a secondary amino acid having an optionally substituted alkyl group;
X7 is an amino acid having an optionally substituted aromatic ring in a side chain;
X8 is an amino acid having an optionally substituted aromatic ring in a side chain;
X9 is any amino acid; and
X10 is any amino acid.

3. The peptide according to claim 2, wherein X1 is an amino acid having an optionally substituted fused ring in a side chain.

4. The peptide according to claim 2, wherein X1 is Nal1, W6N, W7N, or W.

5. The peptide according to any one of claims 2 to 4, wherein X2 is V, K, KCOpipzaa, KCOmeglumine, or Q.

6. The peptide according to any one of claims 2 to 4, wherein X2 is V, K, KCOpipzaa, or KCOmeglumine.

7. The peptide according to any one of claims 2 to 6, wherein X3 is Y, 4Py, A, E, F4G, Q,K, F3G, or 3Py.

8. The peptide according to any one of claims 2 to 6, wherein X3 is Y or 4Py.

9. The peptide according to any one of claims 2 to 8, wherein X4 is an amino acid other than a substituted or unsubstituted aromatic or sulfur-containing amino acid.

10. The peptide according to any one of claims 2 to 8, wherein X4 is D, A, Q, K, or E.

11. The peptide according to any one of claims 2 to 10, wherein X5 is an amino acid other than a substituted or unsubstituted aromatic or sulfur-containing amino acid.

12. The peptide according to any one of claims 2 to 10, wherein X5 is D, E, KCOpipzaa, KCOmeglumine, A, Q, or K.

13. The peptide according to any one of claims 2 to 12, wherein X6 is PeG, K, or MeG.

14. The peptide according to any one of claims 2 to 13, wherein X7 is an amino acid having an optionally substituted fused ring in a side chain.

15. The peptide according to any one of claims 2 to 13, wherein X7 is Nal1, W6N, or W.

16. The peptide according to any one of claims 2 to 15, wherein X8 is Y, 4Py, 3Py, or E.

17. The peptide according to any one of claims 2 to 15, wherein X8 is Y or 4Py.

18. The peptide according to any one of claims 2 to 17, wherein X9 is an amino acid other than a substituted or unsubstituted aromatic or sulfur-containing amino acid.

19. The peptide according to any one of claims 2 to 17, wherein X9 is H, A, Q, or E.

20. The peptide according to any one of claims 2 to 19, wherein X10 is an amino acid other than a substituted or unsubstituted aromatic or sulfur-containing amino acid.

21. The peptide according to any one of claims 2 to 19, wherein X10 is V, E, KCOpipzaa, KCOmeglumine, Q, K, or A.

22. The peptide according to any one of claims 1 to 21, wherein the peptide comprises or consists of an amino acid sequence set forth in any one of
SEQ ID NOs: 2 to 33 or
SEQ ID NOs: 2, 4-13, and 15-33 without a C-terminal glycine (G).

23. The peptide according to any one of claims 1 to 22, wherein the peptide is a cyclic peptide.

24. The cyclic peptide according to claim 23, which has a cyclic structure in which a chloroacetylated amino acid is bonded to a cysteine residue included in the peptide.

25. The peptide according to any one of claims 1 to 24, further comprising an additional amino acid residue.

26. The peptide according to any one of claims 1 to 21 and 23 to 25, comprising a linker at a C-terminus thereof.

27. The peptide according to any one of claims 1 to 26, having TGF-β binding activity.

28. The peptide according to any one of claims 1 to 27, having TGF-β antagonist activity.

29. A medical or diagnostic composition comprising the peptide according to any one of claims 1 to 28.

30. A research composition comprising the peptide according to any one of claims 1 to 28, excluding a composition used as an additive to a medium for culturing an organoid.
